(19) Europäisches Patentamt
European Patent Office
Office européen des brevets

(11) EP 1 424 075 B1

(12) **EUROPEAN PATENT SPECIFICATION**

(45) Date of publication and mention of the grant of the patent:
**27.02.2008 Bulletin 2008/09**

(51) Int Cl.:
***A61K 35/74*** *(2006.01)* ***C12N 1/20*** *(2006.01)*
***A23L 1/00*** *(2006.01)*

(21) Application number: **03256350.4**

(22) Date of filing: **09.10.2003**

(54) **Acid and bile-resistant Lactobacillus isolates having the ability to lower and assimilate cholesterol**

Säure und Gallensalz-resistente Lactobacillus Isolate, welche die Fähigkeit besitzen, Cholesterin zu erniedrigen oder zu assimilieren

Lactobacillus resistant aux acide, et sel biliaire, et capable d'assimiler et baisser le taux de cholesterol

(84) Designated Contracting States:
**AT BE BG CH CY CZ DE DK EE ES FI FR GB GR HU IE IT LI LU MC NL PT RO SE SI SK TR**

(30) Priority: **30.10.2002 CN 02146947**

(43) Date of publication of application:
**02.06.2004 Bulletin 2004/23**

(73) Proprietor: **Food Industry Research and Development Institute**
**Hsin-Chu City (TW)**

(72) Inventors:
- **Liu, Yu-Ju**
  **Hsin-Chu City (TW)**
- **Yu, Chin-Chu**
  **Nan-Tse Dist,**
  **Kaoshsiung City (TW)**
- **Tan, Ching-Fen**
  **Hsin-Chu City (TW)**
- **Liao, Chii-Cherng**
  **Hsin-Chu City (TW)**

(74) Representative: **Bizley, Richard Edward et al**
**HLBBshaw**
**Merlin House**
**Falconry Court**
**Baker's Lane**
**Epping, Essex CM16 5DQ (GB)**

(56) References cited:
**EP-A- 0 792 586**

- **USMAN ET AL: "Viability of Lactobacillus gasseri and its cholesterol-binding and antimutagenic activities during subsequent refrigerated storage in nonfermented milk" JOURNAL OF DAIRY SCIENCE, vol. 82, no. 12, December 1999 (1999-12), pages 2536-2542, XP001182644 ISSN: 0022-0302**
- **PLOCKOVA, MILADA ET AL: "The pH tolerance , bile resistance and production of antimicrobial compounds by lactobacilli" POTRAVINARSKE VEDY , 14(3), 165-174 CODEN: POVEEC; ISSN: 0862-8653, 1996, XP009035096**
- **LUCHANSKY J B ET AL: "MOLECULAR CLONING AND DEOXYRIBONUCLEIC ACID POLYMORPHISMS IN LACTOBACILLUS ACIDOPHILUS AND LACTOBACILLUS GASSERI" JOURNAL OF DAIRY SCIENCE, AMERICAN DAIRY SCIENCE ASSOCIATION. CHAMPAIGN, ILLINOIS, US, vol. 74, no. 10, 1 October 1991 (1991-10-01), pages 3293-3302, XP000233423 ISSN: 0022-0302**

EP 1 424 075 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

SEQUENCE LISTING

<110> Liu, Yu-J .
Yu, Chin-Chu
Tan, Ching-Gen
Liao, chii-cherng

<120> Acid- and bile salt-resistant Lactobacillus isolates having the ability to lower and assimilate cholesterol

<130> PE-23185-AM

<160> 8

<170> PatentIn version 3.2

<210> 1
<211> 500
<212> DNA
<213> Lactobacillus gasseri B21T1

<400> 1

```
caggacgaac gctggcggcg tgcctaatac atgcaagtcg agcgagcttg cctagatgaa    60
tttggtgctt gcaccaaatg aaactagata caagcgagcg gcggacgggt gagtaacacg   120
tgggtaacct gcccaagaga ctgggataac acctggaaac agatgctaat accggataac   180
aacactagac gcatgtctag agtttaaaag atggttctgc tatcactctt ggatggacct   240
gcggtgcatt agctagttgg taaggtaacg gcttaccaag gcaatgatgc atagccgagt   300
tgagagactg atcggccaca ttgggactga gacacggccc aaactcctac gggaggcagc   360
agtagggaat cttccacaat ggacgcaagt ctgatggagc aacgccgcgt gagtgaagaa   420
gggtttcggc tcgtaaagct ctgttggtag tgaagaaaga tagaggtagt aactggcctt   480
tatttgacgg taattactta                                               500
```

<210> 2
<211> 500
<212> DNA
<213> Lactobacillus gasseri B21T6

<400> 2

```
caggacgaac gctggcggcg tgcctaatac atgcaagtcg agcgagcttg cctagatgaa    60
tttggtgctt gcaccaaatg aaactagata caagcgagcg gcggacgggt gagtaacacg   120
tgggtaacct gcccaagaga ctgggataac acctggaaac agatgctaat accggataac   180
aacactagac gcatgtctag agtttaaaag atggttctgc tatcactctt ggatggacct   240
gcggtgcatt agctagttgg taaggtaacg gcttaccaag gcaatgatgc atagccgagt   300
tgagagactg atcggccaca ttgggactga gacacggccc aaactcctac gggaggcagc   360
agtagggaat cttccacaat ggacgcaagt ctgatggagc aacgccgcgt gagtgaagaa   420
gggtttcggc tcgtaaagct ctgttggtag tgaagaaaga tagaggtagt aactggcctt   480

tatttgacgg taattactta                                               500
```

<210> 3
<211> 500
<212> DNA
<213> Lactobacillus gasseri C21T1

<400> 3

caggacgaac gctggcggcg tgcctaatac atgcaagtcg agcgagcttg cctagatgaa 60
tttggtgctt gcaccaaatg aaactagata caagcgagcg gcggacgggt gagtaacacg 120
tgggtaacct gcccaagaga ctgggataac acctggaaac agatgctaat accggataac 180
aacactagac gcatgtctag agtttaaaag atggttctgc tatcactctt ggatggacct 240
gcggtgcatt agctagttgg taaggtaacg gcttaccaag gcaatgatgc atagccgagt 300
tgagagactg atcggccaca ttgggactga gacacggccc aaactcctac gggaggcagc 360
agtagggaat cttccacaat ggacgcaagt ctgatggagc aacgccgcgt gagtgaagaa 420
gggtttcggc tcgtaaagct ctgttggtag tgaagaaaga tagaggtagt aactggcctt 480
tatttgacgg taattactta 500

<210> 4
<211> 500
<212> DNA
<213> Lactobacillus gasseri X21B7

<400> 4

caggacgaac gctggcggcg tgcctaatac atgcaagtcg agcgagcttg cctagatgat 60
tttggtgctt gcactaaatg aaactagata caagcgagcg gcggacgggt gagtaacacg 120
tgggtaacct gcccaagaga ctgggataac acctggaaac agatgctaat accggataac 180
aacactagac gcatgtctag agtttgaaag atggttctgc tatcactctt ggatggacct 240
gcggtgcatt agctagttgg taaggtaacg gcttaccaag gcaatgatgc atagccgagt 300
tgagagactg atcggccaca ttgggactga gacacggccc aaactcctac gggaggcagc 360
agtagggaat cttccacaat ggacgaaagt ctgatggagc aacgccgcgt gagtgaagaa 420
gggtttcggc tcgtaaagct ctgttggtag tgaagaaaga tagaggtagt aactggcctt 480
tatttgacgg taattactta 500

<210> 5
<211> 500
<212> DNA
<213> Lactobacillus gasseri B38T38

<400> 5

caggacgaac gctggcggcg tgcctaatac atgcaagtcg agcgagcttg cctagatgaa 60
tttggtgctt gcaccaaatg aaactagata caagcgagcg gcggacgggt gagtaacacg 120

tgggtaacct gcccaagaga ctgggataac acctggaaac agatgctaat accggataac 180
aacactagac gcatgtctag agtttaaaag atggttctgc tatcactctt ggatggacct 240
gcggtgcatt agctagttgg taaggtaacg gcttaccaag gcaatgatgc atagccgagt 300
tgagagactg atcggccaca ttgggactga gacacggccc aaactcctac gggaggcagc 360
agtagggaat cttccacaat ggacgcaagt ctgatggagc aacgccgcgt gagtgaagaa 420
gggtttcggc tcgtaaagct ctgttggtag tgaagaaaga tagaggtagt aactggcctt 480
tatttgacgg taattactta 500

<210> 6
<211> 480
<212> DNA
<213> Lactobacillus acidophilus B6T7

<400> 6

cagacgaacg ctggcggcgt gcctaataca tgcaagtcga gcgacctgaa ccaacagatt 60
cacttcggtg atgacgttgg gaacgcgagc ggcggatggg tgagtaacac gtggggaccc 120
tgccccatag tctgggatac cacttggaaa caggtgcaat accggataag aaagcagatg 180
ccatgatcag cttataaaag gcggcgtaag ctgtcgctat gggatggccc cgcggtgcat 240
tagctagttg gtagggtaac ggcctaccaa ggcaatgatg catagccgag tttgagagac 300
tgatccggcc acattgggac tgagacacgg cccaaactcc tacgggaggg caagcagtag 360
ggaatcctcc acaatggacc aaagtcctga tggagcaacg ccccgtgagt tgaagaagtt 420
ttcggatcgt aaagccctgt tgttggtgaa gaaggataga ggtaagaact ggcctttatt 480

<210> 7
<211> 480
<212> DNA
<213> Lactobacillus acidophilus

<400> 7

caggacgaac gctggcggcg tgcctaatac atgcaagtcg agcgagctga accaacagat 60
tcacttcggt gatgcagttg ggaacgcgag cggcggatgg gtgagtaaca cgtggggaac 120
ctgccccata gtctgggata ccacttggaa acaggtgcaa taccggataa gaaagcagat 180
gccatgatca gcttataaaa ggcggcgtaa gctgtcgcta tgggatggcc ccgcggtgca 240
ttagctagtt ggtagggtaa cggcctacca aggcaatgat gcatagccga gttgagagac 300
tgatcggcca cattgggact gagacacggc ccaaactcct acgggaggca gcagtaggga 360
atcttccaca atggacgaaa gtctgatgga gcaacgccgc gtgagtgaag aaggttttcg 420
gatcgtaaag ctctgttgtt ggtgaagaag gatagaggta gtaactggcc tttatttgac 480

<210> 8
<211> 480

<212> DNA
<213> Lactobacillus plantarum

<400> 8

```
caggacgaac gctggcggcg tgcctaatac atgcaagtcg aacgaactct ggtattgatt     60
ggtgcttgca tcatgattta catttgagtg agtggcgaaa tggtgagtaa cavgtgggaa    120
acctgcccag aagcggggga taacacctgg aaacagatgc taataccgca taacaacttg    180
gaccgcatgg tccgagttga aagatggctt cggctatcac ttttggatgg tcccgcggcg    240
tattagctag atgctggggt aacggctcac catggcaatg atacgtagcc gacctgagag    300
ggtaatcggc cacattggga ctgagacacg gcccaaactc ctacgggagg cagcagtagg    360
gaatcttcca caatggacga aagtctgatg gagcaacgcc gcgtgagtga agaagggttt    420
cggctcgtaa aactctgttg ttaaagaaga acatatctga gagtaactgt tcaggtattg    480
```

## Description

The present invention relates to novel gastric acid- and bile salt-resistant *Lactobacillus* isolates having the ability to lower and assimilate serum cholesterol and the to use thereof.

"Lactic acid bacteria" are a group of bacteria that can ferment saccharides and that produce lactic acid as a major product. The generally accepted morphological and physiological characteristics of lactic acid bacteria reside in that they are: (1) Gram positive; (2) rod-shaped or disk-shaped; (3) catalase-negative; (4) capable of converting metabolized glucose into more than 50% of lactic acid; (5)nonsporeforming; (6)nonmotile; and (7) microaerobic.

Up to 1980, lactic acid bacteria were known to include, in general, four genera, namely, *Lactobacillus, Streptococcus, Leuconostoc* and *Pediococcus* (W.C. Frazier and D.C. Westhoff, 1978, Food Microbiology, 3rd ed. McGraw-Hill, Inc., New York, USA). In a broader sense, lactic acid bacteria further comprise two genera of *Bifidobacterium* and *Sporolactobacillus.* In recent years, microorganisms have been explicitly classified as a taxonomic group according to DNA homology and rDNA sequence comparison and analysis in the classification system, and have been given a position in taxonomy. To Applicants' knowledge, the family of lactic acid bacteria has expanded to include 16 genera and 223 species by December 1999.

The so-called "probiotics" are live bacteria of a single type or a mixture of bacteria of different types which, after being ingested by the human or animal host, can improve gastrointestinal microbial balance in the human or animal intestinal tract (O'sullivan et al. (1992), Trends in Food Sci. Technol., 3:309-314; Fuller, R., P.J. Heidt, V. Rush and D. van der Waaij. (eds.) (1995), Probiotics: prospects of use in opportunistic infections. Old Herbom University Seminar Monograph No. 8, pp.1). *Lactobacillus* and *Bifidobacterium* are the most widely known and used probiotics.

In 1908, Dr. Eli Metchnikoff proposed the theory that the consumption of large amounts of yogurt containing strains of *Lactobacillus sp.* would result in the replacement of toxic-producing bacteria normally present in the intestine, and would thus result in longevity-without-aging (Eli Metchnikoff, 1908, The Prolongation Of Life, Ed. P. Chalmers Mitchell, G.P. Putnam's Sons, The Knickerbocker Press, New York & London). Recent studies and clinical tests have also shown that *Lactobacillus* is critically related to health. Therefore, *Lactobacillus* has received wide attention in recent years.

Lactic acid bacteria not only play an important role in the complex biosystem of the intestines, they also have probiotic effects on the host. The recognized effects of lactic acid bacteria include: improving nutritional value of host feed; promoting the synthesis of vitamins and the production of enzymes (J. Denter and B. Bisping, 1994, Int. J. Food Microbiol., 22:23-31), inhibiting the growth of intestinal pathogenic bacteria and maintaining the balance of normal intestinal microflora (Hose, H. and Sozzi, T. (1991), J. Chem. Technol. And Biotech. 51:540-544), producing antibodies to enhance immunity of the host (H. Majamaa et al. (1995), Journal of Pediatric Gastro-enterology and Nutrition, 20: 333-338), reducing the risk of colon cancer, and suppressing tumor formation (E.J. Schiffrin et al., 1997, Am. J. Clin. Nutr., 66:515S-520S). Researches also show that consumption of milk fermented with *Lactobacillus* can lower serum cholesterol levels.

Cardiovascular disease has been indicated as a major leading cause of death in industrial countries. In the United States, more people died of coronary heart disease than those of cancer and other diseases, and about three-quarters of the deaths are caused by atherosclerosis and complications thereof. High serum cholesterol level is one of the causes of cardiovascular diseases and atherosclerosis (Kannel et al. (1979), Ann. Intern. Med., 90:85-91; Pekkanen et al. (1990), New England J. Med., 322: 1700-1707). In Taiwan, cerebrovascular and cardiovascular diseases are on the list of top ten leading causes of death, and are the major causes of illness and death in old people. Therefore, hypercholesterolemia is a cause of illness that should not be ignored.

In 1974, Mann and Spoerry found that serum cholesterol levels in men decreased after consumption of yogurt fermented with a *Lactobacillus* strain, whereas no significant change was observed in those that consumed fresh milk (Am. J. Clin. Nutr., 27:464-469, 1974), which spurred extensive researches on the application of lactic acid bacteria and their effects in lowering cholesterol.

K.K. Grunewald reported in J. of Food Science:47:2078-2079(1982) that the serum cholesterol level in rats could be significantly decreased when the rats were given feeds containing 10% milk fermented by *Lactobacillus acidophilus* for four weeks. It was also reported by Danielson et al. in J. Anim. Sci.: 67:966-974(1989) that *acidophilus* yogurt reduced serum cholesterol and low density lipoproteins (LDLs) in boars fed with a high-cholesterol diet in combination with yogurt fermented with *Lactobacillus acidophilus* LA16 isolates for a period of 56 days, but had no effect on serum triglycerides and high density lipoproteins (HDLs).

The aforesaid report, which is related to an isolate strain of *Lactobacillus acidophilus,* LA16, is one of the many works by the research group headed by Dr. Khem M. Shahani on the characteristics and bioactivities of *Lactobacillus acidophilus.* Specifically, Dr. Shahani's group had conducted fairly extensive researches on the characteristics and bioactivities of a specially isolated and cultured strain of *Lactobacillus acidophilus* DDS-1 of human origin, including studies on its effect in reducing serum cholesterol level. The DDS-1 isolated strain was later patented (information regarding DDS-1™ is available on the website of Nebraska Cultures, Inc., http://www.nebraskacultures.com.benefits.html).

In 1997, Akalin et al. compared the effects of ordinary yogurt (fermented by *Streptococcus thermophilus* and *Lactobacillus delbrueckii ssp. bulgaricus*) and *acidophilus* yogurt (fermented by *Streptococcus thermophilus* and *Lactobacillus acidophilus)* on serum cholesterol levels in mice, and found that the ability of *acidophilus* yogurt to reduce serum cholesterol concentrations was significantly higher than that of the ordinary yogurt *(*A.S. Akalin et. al. (1997), J. Dairy Sci., 80:2721-2725).

De Smet et al. reported in British J. of Nutrition, 79:185-194 (1998), that the fecal output of bile salts in pigs fed with *Lactobacillus acidophilus* for four weeks (from week 3 to week 7) increased significantly, and the total serum cholesterol concentrations in the pigs also decreased significantly. It was therefore concluded that the enzymatic activity of bile salt hydrase (BSH, E.C.3.5.1.24) within *Lactobacillus* might be the mechanism responsible for lowering serum cholesterol in the treated pigs.

Cholesterol in humans can be synthesized by the liver, and can also be ingested from meat. There are two pathways for the excretion of cholesterol: (1) forming cholic acid as a result of liver metabolism, which is then conjugated with glycine or taurine to become water-soluble and to subsequently form bile salts such as glycocholates or taurocholates, with potassium or sodium ions for excretion in feces; and (2) forming steroid hormones that are excreted in urine as a result of hormone metabolism; however, only a small portion of the cholesterol is excreted in this manner.

Bile salts are water-soluble final products in cholesterol metabolism. These salts may enter the enterohepatic circulation and, because of the enzymatic activity of bile salt hydrase (BSH) of enteric bacteria, including *Lactobacillus, Enterococcus, Peptostreptococcus, Bifidobacterium, Clostridium* and *Bacteroid, etc*., cholic acids can be separated from glycine or taurine to yield deconjugated bile salts. The deconjugated bile salts do not dissolve in water, and are likely to co-precipitate with serum cholesterol for excretion out of the body.

In addition to the aforesaid, it is reported that the metabolism mechanism of cholesterol includes both assimilation and co-precipitation.

In 1985, it was pointed out that *Lactobacillus acidophilus* could adhere to and assimilate cholesterol, and that, in a medium containing 0.3% oxgall, *Lactobacillus acidophilus* could achieve a higher cholesterol reduction (S.E. Gilliland et al. (1985), Appl. Environ. Microbiol, 49: 377-381). Likewise, it was reported by Noh et al. in J. Dairy Sci. (1997), 82: 3107-3113 that *Lactobacillus acidophilus* was able to incorporate cholesterol into cellular membranes, and the incorporated cholesterol could be further assimilated and metabolized to form substances required by the cells.

On the other hand, it was reported by F.A.M. Kalver and R. van der Meer in Appl. Environ. Microbiol. (1993), 59: 1120-1124 that *Lactobacillus* and *Bifidobacterium* could not assimilate cholesterol, but were able to reduce cholesterol contents in a medium by increasing the conjugating activity of bacterial bile salts when the pH value was below 6.0 to result in co-precipitation of cholesterol with bile salts (this phenomenon may be related to the BSH activity of the bacteria).

In 1997, M.M. Brashears and S.E. Gililland indicated that *Lactobacillus* exhibited good cholesterol co-precipitation during growth without pH control (*i.e.*, at a normal pH of 4.5 to 5.5). However, if the pH value was maintained at about 6.0, the ability of said bacteria to remove cholesterol is significantly decreased (M.M. Brashears and S.E. Gililland (1997), Influences of pH during growth on removal of cholesterol from MRS broth by Lactobacillus casei and Lactobacillus acidophilus, Animal Science Research Report, pp.32-37; http://www.ansi.okstate.edu/research/1997rr/006.htm).

In 1998, Zhang Jia-cheng *et al*. tried to prove that "assimilation" is the major mechanism in the reduction of cholesterol by lactic acid bacteria by experimenting with high lipid milk and edible oil (Zhang Jia-cheng et al. (1998), "The research of cholesterol elimination in food by lactic acid bacteria - the screening of lactic acid bacteria species (strains)," Food Science (P.R.O.C), 19:20-22).

In 1999, Usman and Hosono indicated in J. Dairy Sci., 82: 243-248 that a newly found *Lactobacillus* species, *Lactobacillus gasseri*, can take up cholesterol under culture conditions without bile salts. In another study, the same authors measured the viability of this species in storage *(*J. Dairy Sci., (1999) 82: 2536-2542).

Using a biological method to reduce serum cholesterol levels in humans will be more economical and effective. It is apparent from the aforesaid references that lactic acid bacteria may exhibit the effect of reducing cholesterol both *in vivo* and *in vitro*, and the possible mechanisms include deconjugation by bile salt hydrase (BSH), co-precipitation of cholesterol with deconjugated bile salts under acidic conditions, and assimilation of cholesterol by lactic acid bacterial cells. EP 0 792 586 describes food products containing bacteria with BSH activity.

However, after being ingested into the human body, lactic acid bacteria encounter pressure from the gastrointestinal environment and specificity of intestinal absorption. Therefore, lactic acid bacteria have to first overcome the unfavorable environment of the digestive system and colonize the intestinal tract in order to grow and exert their reaction (s) in the intestinal tract. Further, *Lactobacillus acidophilus* strains are a group of bacteria having complex nutritional requirements. The bacteria are relatively stable in fermented milk. However, the bacteria in commercially available products (in forms of dry powder, grains, tablets) can hardly remain viable after long-term storage at room or refrigerated temperature so that the level of the bacteria cannot be easily maintained at an initial storage level. Accordingly, the actual number of bacteria in a non-fermented milk product is often less than that indicated on the product label. Hence, how to maintain the level of lactic acid bacteria in lactic acid bacteria products during marketing and storage is of utmost importance to manufacturers. Furthermore, screening bacteria to obtain bacterial strains having good resistance to acids and bile salts and capable of lowering serum cholesterol is an important subject in the development of excellent *Lactobacillus* products.

As mentioned above, if resistance to acids and bile salts and storage stability are taken into account when screening bacteria, cost savings can be achieved in the subsequent manufacturing process, and the screened bacterial strains can have a wider range of applications. In addition, the screening for effective bacterial strains is also related to origin and locality. Therefore, in recent years, investigators' efforts have focused on the screening of bacterial strains of human origin.

In Japan, in a total of 171 FOSHU (Foods for Specified Health Use)-approved products, 36 contain probiotic bacteria. They make up about 21% of the total number of products, but have a production value of up to 82%. In Europe, the production value of probiotic bacteria in the food market amounts to 1 billion US dollars. In the United States, yogurt sales in 2000 were up to 1.86 billion US dollars. In Taiwan, the probiotic bacteria market grew to NT$ 4.2 billion dollars in 2000. The applications of probiotic bacteria have increased every year and are no longer restricted to fermented milk, milk, ice cream, candies, and dietary supplements. The products are consumed by adults, infants, poultry, and livestock. It is anticipated that there is huge room for growth of the probiotic market.

There are a number of studies on lactic acid bacteria worldwide. Many of the patents and publications on the acid tolerance and cholesterol lowering ability of *Lactobacillus sp.* are directed to *Lactobacillus acidophilus,* and are largely concerned with bile- and acid-resistant bacterial strains, or are mainly focused on their ability to reduce cholesterol and bile tolerance. At present, studies carried out on the bile tolerance of *Lactobacillus sp.* emphasize that the bacterial strains can grow in an environment containing 0.3% glycocholate, and acid tolerance is tested using media at pH 2, the acidic condition occurring in the gastrointestinal tract at the initial stage of gastric juice secretion. Luchansky et al., (J. Dairy Sci., 74: 3293-3302) reported molecular cloning and deoxyribonucleic acid polymorphisms in *L. acidophilus* and *L. gasseri.*

The ability of *Lactobacillus sp.* to reduce serum cholesterol is about 20% according to published patents, and is in the range from about 10% to about 80%, depending on the methods used, according to publications.

USP 4,839,281 and USP 5,032,399 disclose a strain, *L. acidophilus* GG (ATCC 53103), which was isolated from human feces. This strain can grow in an environment containing 0.15% bile salts, and the amount of residual bacteria after being cultured for 2 hours at pH 1-2 is $10^3$ CFU.

It was reported by S.E. Gilliland and D.K. Walker in J. Dairy Sci.: 73:905-911 (1990) that *L. acidophilus* ATCC 43121 (corresponding to CCRC 17064) of pig origin and *L. acidophilus* ATCC 4356 (corresponding to CCRC 10695) of human origin can both grow in MRS broth supplemented with 0.3% bile salts and have the ability to reduce serum cholesterol.

Further, *L. acidophilus* LA16, the strain isolated from pigs as reported by Danielson et al. in J. Anim. Sci. (1989), 67:966-974*,* and *Lactobacillus acidophilus* DDS-1, an isolated strain of human origin (an endogenous human strain), which was developed by scientists headed by Dr. Khem M. Shahani and which is now a patented product of Nebraska Cultures, Inc., have demonstrated the ability to reduce serum cholesterol (http://www/nebraskacultures.com./benefits.html).

Usman and Hosono reported in J. Dairy Sci., 82: 243-248 (1999) that an isolated *Lactobacillus* strain *Lactobacillus gasseri* exhibited resistance to acids and bile salts and the ability to reduce cholesterol. Plocková *et al.,* also studied the pH tolerance and bile resistance of *Lactobacilli* (Potrav. Védy, 14(3), 165-174).

In USP 5,516,684 and USP 5,707,854, Yoshio Saito and Jun Mizutani disclose two strains of *L. acidophilus,* namely, *L. acidophilus* FERM-P-14204 and *L. acidophilus* FERM-P-14205. These strains do not exhibit deconjugation of bile acids and do not inhibit nutrient absorption, but they demonstrate the lowering of cholesterol in blood and liver.

However, the aforesaid *Lactobacillus* strains are of foreign origin. It would be desirable to obtain *Lactobacillus* isolates which are acid- and bile-resistant and which are capable of lowering serum cholesterol by screening bacteria strains in Taiwan so that the isolates thus obtained can adapt to the gastrointestinal environment of the people of Taiwan and, when used as starters or when added to processed products, can reach the intestine and colonize the intestine after ingestion to thereby enhance the functionality of the products.

Accordingly, in the first aspect, the present invention provides an isolated strain of *Lactobacillus sp.,* wherein the strain is:

(i) a deposited strain selected from:

(a) *Lactobacillus gasseri* B21T1 deposited in the American Type Culture Collection under accession number ATCC PTA-4483;
(b) *Lactobacillus gasseri* B21T6 deposited in the ATCC under accession number ATCC PTA-4484;
(c) *Lactobacillus gasseri* C21T1 deposited in the ATCC under accession number ATCC PTA-4479;
(d) *Lactobacillus gasseri* X21 B7 deposited in the ATCC under accession number ATCC PTA-4480;

(e) *Lactobacillus gasseri* B38T38 deposited in the ATCC under accession number ATCC PTA-4481; or
(f) *Lactobacillus acidophilus* B6T7 deposited in the ATCC under accession number ATCC PTA-4482;

(ii) the sub-cultured offspring of the deposited strain (i); or
(iii) a mutant derived from the deposited strain (i);

wherein the sub-cultured offspring (ii) or mutant (iii) exhibits the bacteriological characteristics of:-

a population decrease of about 0.5 to 2 log values after growth in an environment containing 0.3% oxgall for 24 hours;
an acid tolerance as exhibited by a 3 to 4 log value population decrease after treatment at pH 2 at 37°C for 2 hours, in a 0.85% NaCl/0.01 N HCl system;
the ability to decrease cholesterol content by 93% or higher after anaerobic culture in broth containing 0.3% oxgall and 12% horse serum for 24 hours;
the ability to decrease cholesterol content by 25 to 45% after anaerobic culture in broth containing 0.3% oxgall and 12% cholesterol micelles for 24 hours; and
a cholesterol assimilation rate of 12 to 40%.

In the present invention, feces specimens from healthy infants were used as origins of the bacterial strains. The specimens were screened to isolate bacterial strains capable of resisting acids and bile salts in the gastrointestinal environment and lowering cholesterol. As a result, 6 novel *Lactobacillus* isolates having the aforementioned characteristics were obtained, namely, *Lactobacillus gasseri* B21T1, B21T6, C21T1, X21B7 and B38T38, and *L. acidophilus* B6T7. These isolates were deposited in the Food Industry Research and Development Institute (FIRDI, 331 Shih-Pin Road, Hsinchu City 300, Taiwan, R.O.C.) on June 18, 2002, under Accession Nos. CCRC 910195, CCRC 910196, CCRC 910198, CCRC 910199, CCRC 910197 and CCRC 910194, respectively. These isolates were also deposited in the American Type Culture Collection (ATCC, P.O. Box 1549, Manassas, VA 20108, USA) under the Budapest Treaty on June 21, 2002, and were given ATCC Accession Nos. PTA-4483, PTA-4484, PTA-4479, PTA-4480, PTA-4481 and PTA-4482, respectively.

The novel *Lactobacillus* isolates according to the present invention exhibited a higher cholesterol-lowering ability, as compared to previously known *L. acidophilus* ATCC 43121, ATCC 4356 and DDS-1.

In the second aspect, the present invention provides a composition comprising at least one of the novel *Lactobacillus* isolates according to the present invention or its sub-cultured offspring or a mutant derived therefrom, as claimed in claim 1 and an excipient suitable for the manufacture of foodstuffs, such as beverages, cakes, infant foods, fermented milk, dietary supplements, and animal feed.

According to the third aspect, the present invention provides a pharmaceutical composition comprising at least one probiotically effective amount of one of the novel *Lactobacillus* isolates according to the present invention or its subcultured offspring or a mutant derived therefrom for treating and preventing gastrointestinal diseases as well as lowering serum cholesterol.

The above and other objects and features of the present invention will become apparent with reference to the following description of the preferred embodiments taken in conjunction with the accompanying drawings, in which:

Figure 1 is a bar diagram showing an acid tolerance comparison between the *Lactobacillus* isolates according to the present invention and known *Lactobacillus* strains, in which the acid tolerance is expressed as Δ log (a-b), wherein "a" stands for the number of viable cells after saline treatment at pH 7 for 2 hours, and "b" stands for the number of viable cells after saline treatment at pH 2 for 2 hours;
Figure 2 is a bar diagram showing a bile salt tolerance comparison between the *Lactobacillus* isolates according to the present invention and known *Lactobacillus* strains, in which the bile salt tolerance is expressed as Δlog (c-d), wherein "c" stands for the number of viable cells after incubation in MRS broth for 24 hours, and "d" stands for the number of viable cells after incubation in MRS broth supplemented with 0.3% oxgall for 24 hours;
Figure 3 is a bar diagram showing a growth comparison between the *Lactobacillus* isolate according to the present invention and known *Lactobacillus* strains, both of which were grown in MRS broths with 0.3% oxgall;
Figure 4 shows the relationship between the concentration of cholesterol and the amount of phosphatidycholine-cholesterol micelles, said micelles being prepared with reference to S. Razin et al. (1980), Biochimica Biophysica Acta, 598:628-640, for the purpose of testing the cholesterol-lowering ability of the isolated bacterial strains according to the present invention;
Figure 5 shows the nucleotide sequence of the 16S rDNA of a bacterial isolate according to the present invention, *Lactobacillus gasseri* B21T1;
Figure 6 shows the nucleotide sequence of the 16S rDNA of a bacterial isolate according to the present invention, *Lactobacillus gasseri* B21 T6;

Figure 7 shows the nucleotide sequence of the 16S rDNA of a bacterial isolate according to the present invention, *Lactobacillus gasseri* C21T1;

Figure 8 shows the nucleotide sequence of the 16S rDNA of a bacterial isolate according to the present invention, *Lactobacillus gasseri* X21 B7;

Figure 9 shows the nucleotide sequence of the 16S rDNA of a bacterial isolate according to the present invention, *Lactobacillus gasseri* B38T38; and

Figure 10 shows the differences in the 16S rDNA nucleotide sequences amongst the *Lactobacillus acidophilus* B6T7 isolate of the present invention, *L. acidophilus,* and *L. plantarum,* in which sites of nucleotide differences between the B6T7 isolate and *L. acidophilus* are indicated by bold letters and sites of nucleotide differences between the B6T7 isolate and *L. plantarum* are indicated by the sign "*".

In order to obtain *Lactobacillus* strains indigenous to Taiwan and suitable for growth in the gastrointestinal environment of the people of Taiwan, the Applicants used feces of healthy infants aged one to six years living in Hsinchu City, Taiwan as origins for screening desired bacterial strains, and utilized a series of Rogosa agar-based selective media to screen *Lactobacillus*-suspected strains. 400 suspected strains were found in 828 isolates obtained from 43 specimens. These suspected strains were further tested for the characteristics mentioned in the foregoing Summary of the Invention above, which can be summarized as:

1. Stability to acid;
2. Stability to bile salts; and
3. Ability to reduce cholesterol.

These suspected strains were compared with known strains in terms of the aforesaid characteristics, and were screened to obtain 6 *Lactobacillus* isolates having the desired abilities.

The 6 isolates obtained were further identified using the API identification system, Micro-IS System and 16S rDNA sequence analysis. According to the identification results, these 6 isolates were respectively classified and designated as *Lactobacillus gasseri* 821T1, B21T6, C21T1, X21B7 and B38T38, and *L. acidophilus* B6T7. These isolates were deposited in the Food Industry Research and Development Institute (FIRDI, 331 Shih-Pin Road, Hsinchu City 300, Taiwan, R.O.C.) on June 18, 2002, under the Accession Nos. CCRC 910195, CCRC 910196, CCRC 910198, CCRC 910199, CCRC 910197 and CCRC 910194, respectively. These isolates were also deposited in the American Type Culture Collection (ATCC, P.O. Box 1549, Manassas, VA 20108, USA) under the Budapest Treaty on June 21, 2002, and were given Accession Nos. PTA-4483, PTA-4484, PTA-4479, PTA-4480, PTA-4481 and PTA-4482, respectively.

In view of the beneficial characteristics mentioned above, the *Lactobacillus* isolates according to the present invention are suitable for use as probiotics. For instance, these isolates can be formulated into a broad range of edible materials, including fluid milk (milk, concentrated milk), fermented milk (yogurt, sour milk, frozen yogurt, lactic acid bacteria-fermented beverages), milk powder, ice cream, cream cheeses, dry cheeses, soybean milk, fermented soybean milk, vegetable-fruit juices, fruit juices, sports drinks, confectionery, candies, infant foods, nutritional food products, animal feeds, and dietary supplements. The bacterial count in each of these products may be about $10^6$ to $10^9$ CFU (colony forming units) per gram or per milliliter.

It is apparent to those skilled in the art that the *Lactobacillus* isolates of this invention can be used as a food additive component and, according to known methodologies, they may be added during the preparation of raw materials, or, if their participation in fermentation is not desired, they may be added subsequent to the fermentation process, so as to be formulated into forms suitable for ingestion by humans and animals. Preferably, the *Lactobacillus* isolates of the present invention may be formulated into edible materials alone or in combination with at least one other probiotic organism. These probiotic organisms may include *Lactobacillus sp.,* such as *L. acidophilus, L. lactis, L. brevis, L. casei, L. plantarum, L. salivarius, L. bifidus, L. bulgaricus, L. causasicus* and *L. rhamnosus*; *Streptococcus sp.,* such as *Streptococcus thermophilus,* and *Streptococcus lactis*; yeasts, such as *Candida Kefyr*, and *Saccharomyces florentinus;* or a combination thereof.

In another application, the *Lactobacillus* isolates of the present invention per se or the above-mentioned products incorporating these isolates are prepared as lyophilized powder or spray dry powder such that each product contains about $10^8$ to more than $10^9$ CFU of active *Lactobacillus* cells. Moreover, such products can be prepared in tablet or capsule form by adding thereto yeast powder, saccharides or other filling agents, e.g. digestion improving drugs or instant foods containing *Lactobacillus,* and bacterial powder for direct consumption.

In addition, the present invention also contemplates the use of the aforesaid *Lactobacillus* isolates alone or in combination with other active ingredients as a medicament in controlling the colonization of undesirable intestinal microorganisms in the alimentary tract of a mammal so as to alleviate gastrointestinal problems caused by these undesirable intestinal microorganisms. The composition of the medicament can be formulated into solution, emulsion, powder, tablet, capsule or other suitable forms for oral administration.

Further, in view of the ability of the *Lactobacillus* isolates of the present invention to reduce cholesterol, it is also contemplated that the *Lactobacillus* isolates of the present invention can be used in the preparation of health food products and non-prescription drugs for reducing serum cholesterol.

As described hereinabove, bacterial strains having bacteriological characteristics identical to those of the *Lactobacillus* isolates of the present invention, their sub-cultured offspring, or mutants derived therefrom as claimed in claim 1 are contemplated to fall within the scope and technical concept of the present invention.

As used herein, the term "mutant" refers to a bacterial strain whose genetic composition differs from that of a reference or parent strain by at least one nucleotide due to, e.g. nucleotide substitution, insertion or deletion. A mutant of the invention can be produced by a number of methodologies other than natural mutation. For example, the mutant can be obtained from the random mutagenesis of its parent strain by means of, e.g. chemical mutagen, transposon or irradiation. In addition, a mutant strain of the present invention can include a recombinant nucleic acid sequence. For example, a mutant may be a bacterial strain that harbors an additional nucleic acid sequence, e.g., a sequence transformed, transduced or otherwise inserted into a cell of its parent strain. The additional nucleic acid sequence can encode a polypeptide that is generally or conditionally expressed. Alternatively, the additional nucleic acid sequence can encode a nucleic acid sequence capable of altering cellular physiology, e.g. an anti-sense, a ribozyme, or any other nucleic acid sequence. In another example, the nucleic acid is inserted into an endogenous gene to thereby alter (enhance or destroy) the function of said endogenous gene. For example, the inserted nucleic acid can be a knockout construct that inactivates the endogenous gene, or an artificial enhancer or promoter that increases the transcription of the endogenous gene.

The present invention will be described in more detail with reference to the following examples, which are given for the purpose of illustration only and are not intended to limit the scope of the present invention.

**Example 1 Isolation and screening of *Lactobacillus* isolates**

Materials and Methods:

I. Culture media and Diluents:

1. Screening culture media:

(A) Tomato juice agar medium:

| | |
|---|---|
| Casein enzymatic hydrolysate (Sigma, Louis, Mo, USA) | 10 g |
| Skim milk | 10 g |
| Tomato juice | 400 ml |
| Agar | 12 g |
| Distilled water | adjust to 1L |

(B) Rogosa agar medium:

| | |
|---|---|
| Rogosa agar (Merck,Darmstadt, Germany) | 74.5 g |
| Distilled water | 1 L |

After dissolving agar by microwave heating and cooling the resultant mixture to 55°C, adjust pH to 5.5 with acetic acid.

(C) *Lactobacillus* selective medium (LBS):

| | |
|---|---|
| Rogosa agar | 8.4 g |
| tomato juice | 40 ml |
| distilled water | 60 ml |

After dissolving agar by microwave heating and cooling the resultant mixture to 55°C, adjust pH to 5.5 with acetic acid.

(D) Rogosa + X-glu agar medium:

80 mg of 5-bromo-4-chloro-3-indolyl-β-D-glucopyranoside (X-glu, Sigma, Louis, MO, USA) were added in 100 ml of 0.2 M Tris buffer (pH 8.5), and dissolved using sonification. 10 ml of the resultant solution were added to 190 ml of Rogosa agar at 55°C to form Rogosa + X-glu agar containing X-glu at a final concentration of 40 μg/mL.

2. Bacterial activation medium:

(A) Bacto *Lactobacilli* MRS broth (Difco Laboratories, Detroit, MI, USA); and

(B) MRS agar: Bacto *Lactobacilli* MRS broth supplemented with Agar bacteriological (Scharlau Chemie S.A., Barcelona, Spain)(15 g/L).

3. Ribose utilization medium:

Composition of basal medium:

| | |
|---|---|
| Bacto proteose peptone no.3 (Difco Laboratories, Detroit, MI, USA) | 10 g |
| Bacto yeast extract (Difco Laboratories, Detroit, MI, USA) | 5 g |
| Tween 80 | 1 g |
| Ammonium hydrogen citrate | 2 g |
| Sodium acetate | 5 g |
| Magnesium sulfate | 0.1 g |
| Manganese sulfate | 0.05 g |
| Dipotassium (hydrogen) phosphate | 2 g |
| Chlorophenol red | 0.05% |
| Distilled water | 1 L |
| Adjust pH to 6.3 with HCl. | |

The basal medium as prepared above was packaged into test tubes in an amount of 4.5 ml basal medium per tube. After being sterilized by autoclaving at 121°C for 15 minutes, each tube was added with 0.5 ml of a 10% ribose solution that had been sterilized by filtration.

4. Diluent: 0.1% peptone water (BACTO™ Peptone, Difco Laboratories, Detroit, MI, USA)

II. Procedures to isolate and screen *Lactobacillus* strains:

1. Feces about the size of a finger tip, which were sampled from the middle portion of the feces specimens of infants aged one to six years by using a stick, were added into a test tube containing 9 ml of the above- mentioned diluent (0.1 % peptone water) to yield a 10-fold diluted testing solution. The diluted testing solution was stirred sufficiently and uniformly and was then allowed to stand for several minutes so that microorganisms in the specimens were released into the diluent.
2. 1 ml of the aforesaid diluted testing solution was introduced into another test tube containing 9 ml of the same diluent. A serial dilution was conducted in this manner until a $10^4$-fold dilution was made.
3. $10^2$-, $10^3$- and $10^4$-fold diluted testing solutions, each in an amount of 0.2 ml and prepared by the serial dilution described in step 2, were respectively applied to various screening media (Rogosa agar, tomato juice agar, *Lactobacillus* selective medium and Rogosa + X-glu agar) and were spread evenly by using an L-shape glass rod. These screening media were then placed in an anaerobic incubator containing mixed gases (5% $H_2$, 10% $CO_2$ and 85% $N_2$) set at a temperature of 37 °C and cultured for 2 to 4 days;

4. Rod-shaped bacterial colonies, which exhibited blue color on Rogosa + X-glu agar and which were shown to be non-mobile under microscopic examination, or those which appeared semi-transparent when growing on other screening media and which were also non-mobile under microscopic examination, were selected. The bacteria picked up by a stick were introduced into a test tube containing MRS broth and an inner fermentation tube. After culturing at 37°C for 1 to 2 days, bacterial strains, which proliferated without producing gas and which were shown to have a positive result by Gram-positive staining, were collected.

5. After being activated twice in MRS broth, the bacterial strains collected in step 4 were tested for growth temperature and ribose utilization in the following manners:

(i) MRS broth medium was inoculated with 1% of a bacterial inoculum and was allowed to stand at 15°C for 14 days. If the medium showed to have bacteria growth therein, this indicated that the inoculated bacterial strain can grow at 15°C. On the other hand, if no bacteria growth was found on Day 14, this indicated that the inoculated bacterial strain cannot grow at 15°C.

(ii) MRS broth medium was inoculated with 1% of a bacterial inoculum and was allowed to stand at 45°C for 2 days. If the medium showed to have bacteria growth therein, this indicated that the inoculated bacterial strain can grow at 45°C. On the other hand, if no bacteria growth was found, this indicated that the inoculated bacterial strain cannot grow at 45°C.

(iii) A ribose utilization medium was inoculated with 1% of a bacterial inoculum and was allowed to stand at 37°C for several days.

(iv) If the color of the culture medium turned from purple to yellow, this indicated that the inoculated bacterial strain is able to utilize ribose for growth and produce acid. If the color of the culture medium remained purple after seven days, the medium was allowed to stand a few more days. If the color of the culture medium still did not turn yellow, this indicated that the inoculated bacterial strain is unable to utilize ribose for growth and produce acid.

III. Results:

In this Example, the *Lactobacillus* screening media were primarily based on Rogosa agar, and some of which were further supplemented with tomato juice or X-glu. Tomato juice is rich in Vitamin B groups and is beneficial to the growth of *Lactobacillus.* As for Rogosa agar, the acetic acid contained therein aids in lowering the pH value so as to inhibit bacterial growth, and the high concentration of acetate ions also has the effect of inhibiting the growth of microorganisms.

When a *Lactobacillus acidophilus* strain is cultured on Rogosa agar, it will form white colonies and it is not easily distinguishable from other strains of *Lactobacillus.* If X-glu is added to Rogosa agar, the bacterial colonies of *Lactobacillus acidophilus* would become blue in color. This is because the cells of *Lactobacillus acidophilus* have the activity of β-D-glucosidase which can break up the β -D-glucosidic bond of X-glu to produce a blue chromogene, which makes the bacterial colonies of *Lactobacillus acidophilus* look blue. However, since this enzyme is also present in the cells of many other microorganisms, the selected bacterial strains have to undergo microscopic examination, Gram staining and the above-mentioned physiological and biochemical tests for ribose utilization and growth temperature before suspected strains with the desired properties can be selected.

Applicants collected 43 feces specimens from healthy infants aged one to six years living in Hsinchu City, Taiwan. The diluted sample of each specimen was plated on each of the selective media according to the procedures described above. When formation of bacterial colony on the selective media was observed, bacterial colonies with different colors and morphologies were selected to undergo microscopic examination. Bacteria colonies which were formed by rod-shaped bacterial calls were further subjected to Gram staining. Any bacteria colony that was found to contain Gram-positive rod bacteria was then selected and referred to as an "isolate."

The isolates thus selected were respectively introduced into MRS broth containing an inner fermentation tube therein, and those that do not produce gas during culture were selected to undergo growth temperature and ribose utilization tests. Any tested strain that can grow at 45°C but not at 15°C, and that cannot utilize ribose as carbon source for growth would be stowed aside, and the strains selected at this stage are referred to as "suspected strains."

Referring to Table 1, Applicants obtained 400 suspected strains out of a total of 828 isolates selected from the collected 43 specimens.

Table 1. Numbering of collected specimens* and numbers of bacterial isolates obtained from said specimens

| Specimen number | Numbers of bacterial isolates | Number of suspected strains | Specimen number | Numbers of bacterial isolates | Numbers of suspected strains |
|---|---|---|---|---|---|
| 1 | 40 | 0 | 23 | 3 | 0 |
| 2 | 0 | 0 | 24 | 0 | 0 |
| 3 | 0 | 0 | 25 | 100 | 100 |
| 4 | 26 | 0 | 26 | 0 | 0 |
| 5 | 45 | 0 | 27 | 0 | 0 |
| **6**** | 68 | 55 | 28 | 0 | 0 |
| 7 | 0 | 0 | 29 | 0 | 0 |
| 8 | 0 | 0 | 30 | 60 | 0 |
| 9 | 0 | 0 | 31 | 0 | 0 |
| 10 | 0 | 0 | 32 | 0 | 0 |
| 11 | 0 | 0 | 33 | 0 | 0 |
| 12 | 60 | 60 | 34 | 0 | 0 |
| 13 | 0 | 0 | 35 | 0 | 0 |
| 14 | 0 | 0 | 36 | 139 | 115 |
| 15 | 0 | 0 | 37 | 0 | 0 |
| 16 | 0 | 0 | **38**** | 120 | 40 |
| 17 | 0 | 0 | 39 | 0 | 0 |
| 18 | 0 | 0 | 40 | 0 | 0 |
| 19 | 0 | 0 | 42 | 60 | 0 |
| 20 | 14 | 0 | 42 | 0 | 0 |
| **21**** | 30 | 30 | 43 | 60 | 0 |
| 22 | 3 | 0 | **Total** | **823** | **400** |

*Origin of specimens: feces from infants aged one to six years living in Hsinchu City, Taiwan.
**: The specimen numbers from which the *Lactobacillus* isolates according to this invention were obtained.

The suspected strains were then subjected to acid tolerance test, bile salt tolerance test and cholesterol lowering test which are described in the following Examples. Six isolates, namely, B21T1, B21T6, C21T1, X21B7, B38T38 and B6T7, exhibited excellent properties in these tests and were therefore compared with the following known bacterial strains in terms of the above-indicated properties:

1. *Lactobacillus acidophilus* CCRC 17064 (corresponding to ATCC 43121; isolated from pigs): this *Lactobacillus* strain has the ability to reduce serum cholesterol (S.E. Gilliland et al. (1985), Appl. Environ. Microbiol., 49: 377-381; S.E. Gilliland and D.K. Walker (1990), J. Dairy Sci., 73:905-911; F.A.M. Kalver and R. van der Meer (1993), Appl. Environ. Microbiol, 59: 1120-1124; D.O. Noh et al. (1997), J. Dairy Sci., 82: 3107-3113);
2. *Lactobacillus acidophilus* CCRC 10695$^T$ (corresponding to ATCC 4356; isolated from humans): this *Lactobacillus* strain is a standard strain of *Lactobacillus acidophilus* and is of human origin (D.K. Walker and S.E. Gilliland (1993), J. Dairy Sci., 76:956-961);
3. *Lactobacillus acidophilus* DDS-1 (isolated from humans): this *Lactobacillus* strain is a commercially available product (Nebraska Cultures, Inc.);
4. *Lactobacillus acidophilus* CCRC 14065 [corresponding to CSCO 2401, commercially available from Commonwealth Scientific Industrial Research Organization (CSIRO), Canberra, Australia]; and
5. *Lactobacillus gasseri* CCRC 14619$^T$ (corresponding to ATCC 33323; isolated from humans)(Int. J. Syst. Bacterial. (1980), 30, 601; E. Lauer and O. Kandler, Bakteriol. Parasitenkd. Infektionskr. Hyg. Abt. 1 Orig. Reihe C, 1980, 1, 75-78).

**Example 2 Acid tolerance test**

I. Experimental Procedures:

The bacterial cell survival test conducted in this example was performed with reference to the method described in J.E. Holcombe et al. (1991), Cult. Dairy Prod. J., 26 (3): 4-5 and that disclosed in US 5,711,977 issued to Y.S. Yang *et al.*, in which a neutral environment (pH 7) and an acidic environment (pH 2) that simulated the stomach were employed.

After being activated twice in MRS broth, the culture of each of the tested bacterial strains was centrifuged at 3,000 rpm for 10 min. After removal of the supernatant, the cell pellet was re-suspended in 1 ml of normal saline (0.85% NaCl, pH 7) and stirred by a stick, followed by vortexing to obtain a homogenous bacterial suspension. Aliquots (0.5 ml) of the resultant bacterial cell suspension were admixed with normal saline solutions of pH 7 and pH 2, respectively, and the resultant mixtures were allowed to stand in a 37°C incubator for 2 hours. Thereafter, the bacterial cells that survived were counted, and the acid tolerance of each tested bacterial strain was expressed by Δlog (number of bacterial cells), *i.e.* a calculated log value of (the number of bacterial cells that survived after the normal saline treatment at pH 7 minus the number of bacterial cells that survived after the normal saline treatment at pH 2). Bacterial strains with a smaller value of Δlog (number of bacterial cells) were considered to have higher acid tolerance.

II. Results:

Most microorganisms do not have any tolerance in an acidic environment. Although lactic acid bacteria per se are acid-producing bacteria, they only grow in environments with pH 3.2 to 4.5. Therefore, the extremely acidic environment of the stomach (pH 2.0 to 3.2) is also a major factor affecting the survival rate of the lactic acid bacteria. Depending on the entry time and type of the gastric contents, the pH value of gastric acid will vary in a range from pH 1.5 to 4.5 during a period of 2 hours in average. Therefore, in the experiment of this example, pH 2 was used as a representative of the gastric pH value. Furthermore, since gastric acid is similar to hydrochloric acid in nature, normal saline with pH adjusted to 2 by hydrochloric acid (0.85% NaCl/0.01 N HCl) was used to treat the tested bacterial strains at 37 °C for 2 hours. The bacterial cells that survived in this acid treatment were counted and compared with those of the control group treated with normal saline at pH 7.

The obtained experimental results showed that, after treatments at the above-indicated two different pH values at 37°C for 2 hours, *Lactobacillus acidophilus* CCRC 17064 exhibited the highest acid tolerance (Figure 1), and the bacterial count thereof after acid treatment merely decreased by 1.9 in log value. On the other hand, *Lactobacillus acidophilus* CCRC $10695^T$ and *Lactobacillus gasseri* CCRC $14619^T$ exhibited poor acid tolerance, and their bacterial counts decreased by 4.3 and 4.4 in log value, respectively.

The acid tolerance of the new isolate B21T1 obtained in Example 1 of this invention (which was classified as *Lactobacillus gasseri* according to the bacteriological characteristics thereof, as described in the following Example) is similar to that of CCRC 14065, and their bacterial counts decreased by 3.9 in log value. The other five new isolates of the present invention (B21T6, C21T1, X21 B7, B38T38 and B6T7) have similar levels of acid tolerance, and are able to meet the screening index of acid tolerance, a value of Δlog (number of bacterial cells) < 4 (US 5,711,977 issued to Y.S. Yang et. al.).

**Example 3. Bile salt tolerance test**

I. Experimental Procedures: 1% of a bacterial inoculum

After being activated twice in MRS broth, 1% inoculum of each tested bacterial strain was inoculated into 10 ml MRS broth and 10mL MRS broth supplemented with 0.3% oxgall (MRSO), respectively. After culturing at 37°C for 24 hours, the bacterial density ($OD_{660}$) of each culture was measured by a spectrophotometer, and the number of surviving bacterial cells was also counted. The bile salt tolerance is expressed by Δlog (number of bacterial cells), *i.e.* a calculated log value of (the number of bacterial cells cultured in MRS broth minus the number of bacterial cells cultured in MRSO broth), in which the smaller the calculated Δlog (number of bacterial cells) is, the higher will be the bile salt tolerance of the tested bacterial strain.

II. Results:

Different lactic acid bacteria exhibited vast differences in survival rate after being treated with bile salts. Therefore, screening lactic acid bacteria with bile salt tolerance is important in the selection of probiotic bacteria (S.E. Gilliland et al.(1984), J. Dairy Sci., 67:3045-3051; P. Marteau et al. (1997), J. Dairy Sci., 80:1031-1037). In earlier studies, oxgall was widely used in media for selective culturing of human intestinal bacteria. Therefore, the effectiveness of oxgall should be very similar to that of human bile salts, and oxgall was commonly used in an average concentration of 0.3% (w/v) (S.E. Gilliland and D.K. Walker (1990), J. Dairy Sci., 73: 905-911; D.K. Walker and S.E. Gilliland (1993), J. Dairy Sci., 76: 956-961).

The obtained experimental results showed that, except for CCRC $10695^T$, CCRC $14619^T$ and CCRC 14065, the measured $OD_{660}$ value (bacterial density) of each tested bacterial strain was greater than 2 (Table 2), regardless of whether the tested bacterial strain was cultured in a medium with or without 0.3% oxgall. This indicates that the addition of 0.3% oxgall seems to have little effect upon the growth of the tested bacterial strains.

Table 2. Growth comparison of tested bacterial strains cultured in MRS broth with and without 0.3% oxgall for 24 hours.

| Cultures of tested bacterial strains | Bacterial density ($OD_{660}$ value) | |
|---|---|---|
| | MRS Broth | MRS Broth + 0.3% oxgall |
| B6T7 | 2.49 | 2.74 |
| C21T1 | 2.44 | 2.56 |
| B38T38 | 2.45 | 2.03 |
| B21T1 | 2.57 | 2.53 |
| B21 T6 | 2.62 | 2.29 |
| X21B7 | 2.69 | 2.50 |
| CCRC 17064 | 2.91 | 2.37 |
| CCRC $10695^T$ | 2.35 | 1.09 |
| CCRC 14065 | 2.55 | 1.79 |
| CCRC $14619^T$ | 2.96 | 1.58 |
| DDS-1 | 2.87 | 2.72 |

However, it is also noted that the bacterial densities of two new isolates according to the present invention, namely, B6T7 and C21T1, are higher in MRSO broth than in MRS broth. This shows that other factors may exist that interfere with light absorption. Therefore, the numbers of surviving bacterial cells of the tested bacterial strains cultured in these two media for 24 hours were further examined.

Referring to Figure 2, it can be seen from a reduction in the cell number of bacterial strains examined that, CCRC $14619^T$ has a very poor bile salt tolerance, and CCRC $10695^T$ and the new isolate B38T38 of the present invention were shown to have a bile salt tolerance higher than that of CCRC $14619^T$, respectively. The bacterial strain exhibiting the highest bile salt tolerance is the new isolate B6T7 of the present invention, and the bile salt tolerance of the rest of the bacterial strains being substantially similar. That is, after culturing for 24 hours in MRS broth containing 0.3% oxgall, the numbers of dead bacterial cells thereof range from 1 to 2 in log values.

Further, the growth curves of some bacterial strains cultured in MRS broth containing 0.3% oxgall were observed. The growth rate of each of the tested strains is represented by the gradient of the growth curve. That is to say, the larger the gradient, the faster the growth rate, and thus, the higher the tolerance to bile salt. It can be seen from Figure 3 that the growth rate of CCRC 17064 is the fastest, those of the new isolates X21B7 and B21T1 of the present invention being slower, and those of CCRC 14065, CCRC $10695^T$ and DDS-1 being the slowest.

Although slight differences are present amongst the experimental results obtained from different observation methods, it can be appreciated from these tests that the six new *Lactobacillus* isolates screened in the present invention exhibit same tolerance against acid and bile salts.

**Example 4. Assay for the ability of the tested bacterial strains in lowering cholesterol**

I. Experimental Procedure:

(A) Source of Cholesterol:

In respect to the test of bacterial strains in lowering cholesterol, PPLO (BACT PPLO SERUM FRACTION) was used as the source of cholesterol in most prior references and patent publications. However, production of this product has already ceased. Therefore, in this Example, Applicants adopted the phosphatidycholine-cholesterol preparation methods disclosed in Huang, C. and T.E. Thomopson (1974), Methods Enzymol., 32:485-489 and in S. Razin et al. (1980), Biochimica Biophysica Acta, 598:628-640, in which horse serum and phosphatidycholine-cholesterol were used as the source of cholesterol to prepare a homogeneously distributed phosphatidycholine-cholesterol solution, the volume of which is in a positive linear functional relationship with the cholesterol concentration (Figure 4).

1. Horse serum

Horse serum dissolved in water was filtered through a 0.45 μm membrane filter and was stored in a -20°C refrigerator.

2. Phosphatidycholine-cholesterol (egg lecithin + cholesterol)

According to S. Razin et al. (1980), Biochimica Biophysica Acta, 598:628-640, egg Lecithin and cholesterol were placed into a ground stopper bottle of a rotary vacuum evaporator (EYELA), and were uniformly dissolved in a sufficient amount of chloroform, followed by drying via nitrogen purging so that cholesterol and egg lecithin were mixed homogeneously to form a uniform film on the glass wall of the ground stopper bottle. After re-dissolving the film in a 0.4 M sucrose solution, the ground stopper bottle was charged with $N_2$ gas therein and then sealed with a cap, followed by wrapping with an aluminum foil, so as to reduce the probability of lipid oxidization.

After the sealed ground stopper bottle was sonicated in a water bath-type sonicator at 4°C for 15 minutes, the mixed solution thus formed was passed through a 0.22 μm membrane filter under pressure twice, and was then stored in a 4°C refrigerator (the solution must be used within 3 days of its preparation).

(B) Test grouping:

Test (1): After being activated twice in MRS broth, 1% inoculum of each of the tested bacterial strains was inoculated into 10 ml MRSS containing 8.8 ml MRS, 1.2 ml cholesterol solution as prepared above and 0.3% oxgall, and cultured in a 37°C anaerobic incubator (10% $CO_2$ and 90% $N_2$) for 24 hours. Thereafter, according to the method described in Item (C) below, supernatant samples and cell pellet samples were respectively collected from the resultant cultures inoculated with the tested bacterial strains for measurement of cholesterol content.

Test (2): After being activated twice in MRS broth, 1% inoculum of each of the tested bacterial strains was inoculated into 10 mL MRSO and cultured at 37°C for 20-22 hours. Thereafter, 1% inoculum taken from the resultant culture of each tested bacterial strain was inoculated into 10 ml MRSS and cultured in a 37°C anaerobic incubator (10% $CO_2$ and 90% $N_2$) for 24 hours. Thereafter, according to the method described in Item (C) below, supernatant samples and cell pellet samples were respectively collected from the resultant cultures inoculated with the tested bacterial strains for measurement of cholesterol content.

(C) Measurement of cholesterol content

The measurement of cholesterol content was conducted according to the *o*-phthalaldehyde method (L.L. Rudel and M.D. Morris (1973), Notes on Methodology, 14:364-366; S.E. Gilliland et al. (1985), Appl. Environ. Microbiol, 49: 377-381).

With respect to each of the resultant cultures inoculated with the tested bacterial strains as described in the above Test (1) and Test (2), a tested bacterial solution in an amount of 1 ml was sampled and centrifuged at 12,000 rpm for 10 minutes. 0.5 ml of the supernatant thus formed was placed into a tube to serve as a supernatant specimen of the resultant culture inoculated with a respective tested bacterial strain.

With respect to each of the resultant cultures inoculated with the tested bacterial strains as described in the above Test (1) and Test (2), a tested bacterial solution in an amount of 1 ml was sampled and centrifuged at 3,000 rpm for 10 minutes, and the resultant supernatant was removed. The left cell-pellet was re-dissolved in 10 ml MRSO broth and mixed uniformly. A 0.5 ml solution was taken from the resultant mixture and placed into a test tube to serve as a cell-pellet specimen of the resultant culture inoculated with a respective tested bacterial strain.

Each tested specimen was added with 3 ml of 95% ethanol and mixed well by uniform shaking. 2 ml of 50% KOH was further added thereto, and the resultant mixture was again shaken uniformly. Thereafter, the mixture was heated at 60°C in a water bath for 10 minutes and then allowed to cooled at room temperature. The cooled mixture was added with 5 ml hexane and stirred uniformly, followed by addition of 3ml $H_2O$. After being shaken uniformly, the mixture so formed was allowed to stand at room temperature for 15 minutes. 2.7 ml of the resultant hexane layer was removed into another test tube, and hexane was evaporated at 60°C under $N_2$. The solid residue left after evaporation was added with 4 ml of a *o*-phthalaldehyde reagent solution (0.5 mg *o*-phthalaldehyde/1 ml acetic acid). The resultant mixture was shaken uniformly and allowed to stand at room temperature for 10 minutes, followed by addition of 2 ml concentrated sulfuric acid. The mixture thus formed was shaken uniformly and allowed to stand at room temperature for 10 minutes. Thereafter, the mixture was subjected to an $OD_{550}$ measurement using a spectrophotometer.

(D) Assessment of the ability of a tested bacterial strain in lowering cholesterol

The ability of a bacterial strain in lowering cholesterol was estimated using the following formula:

$$A = 100 - [(B/C) \times 100]$$

A = reduction in cholesterol (%)
B = cholesterol content (mg) in the supernatant of the culture inoculated with the tested bacterial strain
C = cholesterol content (mg) in the supernatant of the culture without inoculation of the tested bacterial strain (control group)

If the value of "A" value is greater than 80%, the bacterial strain is considered to have a notable ability in lowering cholesterol.

(E) Assessment of the ability of a tested bacterial strain in assimilating cholesterol

$$A' = 100 - [(B' + C') \times 100]$$

A' = assimilation of cholesterol (%)
B' = cholesterol content (mg) in the supernatant of the culture inoculated with the tested bacterial strain
C' = cholesterol content (mg) in the cell-pellet of the culture inoculated with the tested bacterial strain

If the value of "A'" value is greater than 15%, the bacterial strain is considered to have a notable ability in assimilating cholesterol.

II. Result:

(i) The Abilities of tested bacterial strains in lowering cholesterol

In comparing Test (1) (*i.e.* culturing a tested bacterial strain in MRS broth) and those of Test (2) (*i.e.* culturing a tested bacterial strain in MRSO broth), the difference therebetween resides in that in Test (2), a tested bacterial strain was inoculated into MRSO broth and cultured for 20 to 22 hours. 1% inoculum was taken therefrom and inoculated into 10 ml MRSS for further culture. The purpose of Test (2) is to screen the tested bacterial strains again to determine whether they have excellent bile salt tolerance, and to also screen bacterial strains having the ability in lowering cholesterol.

The results of the tests using horse serum as cholesterol source (Table 3) showed that prior *Lactobacillus acidophilus* CCRC 17064 and DDS-1 also demonstrated significant effects in this test, and their abilities in lowering cholesterol reached 88 to 98%. On the other hand, the abilities of CCRC 14065, CCRC $10695^T$ and CCRC $14619^T$ in lowering cholesterol were not significant. This might be due to the fact that these bacterial strains have poor bile salt tolerance and do not grow well in MRSO broth so that their cholesterol lowering abilities as observed reached only 30 to 43%.

In these two tests, the new isolates of the present invention, *i.e.* B6T7, C21T1, B21T1, B21T6, B38T38 and X21B7, were shown to have a cholesterol lowering ability up to 91-99% or more, which is slightly higher than those of CCRC 17064 and DDS-1. This shows that the bacterial strains screened in accordance with the present invention also have excellent ability in lowering serum cholesterol.

Table 3. Assessment of the ability of tested bacterial strains in decreasing cholesterol (horse serum model)[a]

| Cultures | Growth in MRS broth[b] | | Growth in MRSO broth[c] | |
|---|---|---|---|---|
| | Cholesterol[d] in spent broth (μg/ml) | Cholesterol decrease (%) | Cholesterol[d] in spent broth (μg/ml) | Cholesterol decrease (%) |
| B6T7 | 6.71 | 92.2 | 7.14 | 92.7 |
| C21T1 | 0.69 | 99.2 | 2.41 | 97.2 |
| B38T38 | 2.24 | 97.4 | 7.57 | 91.2 |
| 821T1 | 3.10 | 96.6 | 0.34 | 99.6 |
| B21 T6 | 2.84 | 96.7 | 2.24 | 97.4 |
| X21B7 | 1.34 | 98.4 | 3.27 | 96.2 |
| CCRC 17064 | 1.12 | 98.7 | 9.81 | 88.4 |
| CCRC 10695 | 8.00 | 90.7 | 56.97 | 33.8 |
| CCRC 14065 | 11.70 | 86.4 | 59.90 | 30.4 |
| CCRC 14619 | 2.84 | 96.7 | 49.14 | 42.9 |
| DDS-1 | 5.94 | 93.1 | 5.16 | 94.0 |
| Control | 86.06 | - | 86.06 | - |

[a]: MRS broth supplemented with 0.3% oxgall and 12% horse serum.

[b]: Before the test, all cultures were sub-cultured in MRS broth using 1% inoculum and incubated at 37°C for 24 h.

[c]: Before the test, all cultures were sub-cultured in MRSO broth (MRS broth with 0.3% oxgall) using 1 % inoculum and incubated at 37°C for 24 h.

[d]: Initial cholesterol content in broth was 86.06 μg/ml.

Referring to Table 4, when phosphatidycholine-cholesterol was used instead of horse serum as cholesterol source in the test in connection with the assessment of the ability of tested bacterial strains in lowering cholesterol, it was observed that the ability of each bacterial strain in terms of cholesterol decrease (%) tends to decrease. This indicates that the ability of *Lactobacillus* strains in lowering cholesterol level may vary with the type of cholesterol in use. It is noted that the ability of CCRC 17064 in lowering phosphatidycholine-cholesterol is 11 to 29%, whereas the abilities of the bacterial strains of the present invention in lowering phosphatidycholine-cholesterol reached 23 to 48%, which is evidently higher than that achieved by CCRC 17064, although slightly lower than that achieved by DDS-1 (50%).

Table 4. Assessment of the abilities of tested bacterial strains in lowering cholesterol (phosphatidylcholine-cholesterol micelle model)[a]

| Cultures | Growth in MRS broth[b] | | Growth in MRSO broth[c] | |
|---|---|---|---|---|
| | Cholesterol[d] (μg/ml) | Cholesterol decrease (%) | Cholesterol[d] (μg/ml) | Cholesterol decrease (%) |
| B6T7 | 45.05 | 23.92 | 33.70 | 43.10 |
| C21T1 | 30.71 | 48.15 | 43.59 | 26.40 |
| B38T38 | 33.75 | 43.01 | 35.16 | 40.62 |
| B21T1 | 32.34 | 45.39 | 32.83 | 44.57 |
| B21T6 | 35.43 | 40.16 | 36.25 | 38.79 |
| X21B7 | 35.11 | 40.71 | 39.29 | 33.65 |
| CCRC 17064 | 52.66 | 11.07 | 42.01 | 29.06 |
| DDS-1 | 29.24 | 50.63 | 29.46 | 50.26 |
| Control | 59.22 | - | 59.22 | - |

[a]: MRS broth supplemented with 0.3% oxgall and 12% phosphatidylcholine- cholesterol micelles.

[b]: Before the test, all cultures were sub-cultured in MRS broth using 1% inoculum and incubated at 37°C for 24 h.

[c]: Before the test, all cultures were sub-cultured in MRSO broth (MRS broth with 0.3% oxgall) using 1% inoculum and incubated at 37°C for 24 h.

[d]: Initial cholesterol content in broth was 59.22 μg/ml.

(ii) Cholesterol assimilation by tested bacterial strains:

Concerning the mechanism of lactic acid bacteria in lowering cholesterol, cholesterol is removed by the co-precipitation of cholesterol and deconjugated bile salts (F.A.M. Kalver and R. van der Meer (1993), *supra,* M.M. Brashears and S.E. Gililland (1997), *supra*), and the assimilation of cholesterol by the bacterial strain per se (Gilliland *et al.* (1985), *supra*; D.O. Noh *et al.* (1997), *supra;* and Zhang *et al* (1998), *supra*).

Referring to Table 5, it can be seen that CCRC 17064, the six isolates obtained according to the present invention, and DDS-1 can assimilate cholesterol. However, different bacterial strains differ slightly in their abilities to assimilate cholesterol. The observed assimilation rate is in the range of from 11 to 40%. Cholesterol assimilation by CCRC 14065, CCRC $10695^T$ and CCRC $14619^T$ was not significant.

Table 5. Assessment of the cholesterol assimilation[a] of tested bacterial strains

| | Growth in MRS broth[b] | | | Growth in MRSO broth[c] | | |
|---|---|---|---|---|---|---|
| Cultures | Cholesterol[d] in spent broth (%) | Cholesterol in cell pellet (%) | Cholesterol assimilated (%) | Cholesterol[d] in spent broth (%) | Cholesterol in cell pellet (%) | Cholesterol assimilated (%) |
| B6T7 | 7.8 | 51.4 | 40.8 | 7.3 | 74.1 | 18.6 |
| C21T1 | 0.8 | 86.0 | 13.2 | 2.8 | 72.3 | 24.9 |
| B38T38 | 2.6 | 79.1 | 18.3 | 8.8 | 83.7 | 7.5 |
| B21T1 | 3.4 | 81.7 | 14.9 | 0.4 | 88.4 | 11.2 |
| B21T6 | 3.3 | 67.2 | 29.5 | 2.6 | 81.4 | 16.0 |
| X21B7 | 1.6 | 67.0 | 31.4 | 3.8 | 71.3 | 24.9 |
| CCRC 17064 | 1.3 | 68.5 | 30.2 | 11.6 | 72.9 | 15.5 |
| CCRC 10695 | 9.3 | 71.7 | 19.0 | 66.2 | 33.4 | 0.4 |
| CCRC 14065 | 13.6 | 82.0 | 4.4 | 69.6 | 27.8 | 2.6 |
| CCRC 14619 | 3.3 | 80.6 | 16.1 | 57.1 | 42.7 | 0.2 |
| DDS-1 | 6.9 | 78.5 | 14.6 | 6.0 | 81.3 | 11.2 |

[a]: MRS broth supplemented with 0.3% oxgall and 12% horse serum.
[b]: Before the test, all cultures were sub-cultured in MRS broth using 1% inoculum and incubated at 37°C for 24 h.
[c]: Before the test, all cultures were sub-cultured in MRSO broth (MRS broth with 0.3% oxgall) using 1 % inoculum and incubated at 37°C for 24 h.
[d]: The concentration of cholesterol in the control was defined as 100 parts.

Summarizing the above test results, it is clear that the six new *Lactobacillus* isolates, obtained and screened in Taiwan in accordance with the present invention, have demonstrated excellent acid- and bile salt-resistance and the ability to lower cholesterol. Further identification and characterization tests of these *Lactobacillus* isolates were conducted in the following examples.

**Example 5. Identification and characterization of *Lactobacillus* isolates**

I. Experimental Procedures:

(1) Preliminary identification tests:

Freshly cultured bacterial strains (cultured for 18 to 24 hours) were subjected to preliminary identification tests, the conducted items of which include: Gram staining, morphological observations, catalase test, motility, growth under aerobic and anaerobic conditions (O. Kandler and N. Weiss (1986), Regular, nonsporing Gram-positive rods and Cocci. In: Bergey's Manual of Systematic Bacteriology. (Sneath, P. H. A., Mair, N. S., Sharpe, M. E., and Hotl, J. G., ed.) Vol. II. pp. 1208-1234. The Williams & Wilkins Co., Baltimore, USA).

(2) API identification system:

This identification test is based on that described in G. H. Fleet et al. (1984), Appl. Environ. Microbiol., 48:

1034-1038. The identity of the lactic acid bacteria was verified with the API 50 CHL Kit (French API BioMerieux Research Laboratory, La Balme Les Grottes, Montalien, Jeraeh, France). The items tested were: tests of acid production from 49 carbon sources that include: glycerin, erythritol, D-arabinose, L-arabinose, ribose, D-xylose, L-xylose, adonitol, β-methyl-xyloside, galactose, D-glucose, D-fructose, D-mannose, L-sorbose, rhamnose, dulcitol, inositol, mannitol, sorbitol, α-methyl-D-mannoside, α-methyl-D-glucoside, N-acetyl glucosamine, amygdalin, arbutine, esculin, salicin, cellobiose, maltose, lactose, melibiose, saccharose, trehalose, inuline, melezitose, D-raffinose, amidon, glycogen, xylitol, β-gentiobiose, D-turanose, D-lyxose, D-tagatose, D-fucose, L-fucose, D-arabitol, L-arabitol, gluconate, 2-keto-gluconate, and 5-keto-gluconate.

The API system works according to the following operating procedures: using a sterile swab to pick up a bacterial colony grown on a culture plate and allowing the picked bacterial colony to be evenly suspended in 2ml of 0.85% normal saline; sucking the bacterial cell-suspended solution using a sterile pipette and dropping n-drops of the solution into another 5 ml of 0.85% sterile normal saline such that the concentration of the resultant bacterial solution is equivalent to that of McFanand No. 2 standard solution (a mixed solution formed from 9.8 ml of 1 % $H_2SO_4$ and 0.2 ml of 1 % $BaCl_2$). In the meantime, an additional bacterial cell-suspended solution is taken, and 2n drops of the solution are pipetted into a glass tube containing API 50 CHL medium. After mixing uniformly, a proper volume of the inoculum so prepared is added into each of the tubes of a test strip, overlaid with mineral oil, and placed in the incubation tray (adding water to the tray beforehand so as to avoid loss of the medium through evaporation during incubation). After being covered with an incubation lid, the incubation tray is allowed to stand at 37°C for 48 hours and is subsequently taken out for reading purposes. The results are recorded and compared with the database of API LAB Software identification system, so as to determine the most appropriate taxonomic genera and species for the bacterial colony under test.

(3) Micro-IS System:

The Micro-IS System is based on the method proposed by M. Rogosa et al (1971), Method for coding data on microbial strains for computers (edition AB), Int. J. Syst. Bacteriol. 21:1A-184A, and utilizes Matrix 5 of Micro-IS System (suitable for *Lactobacillus sp.*). The test items include: motility; growth temperature test (15°C, 45°C); growth under aerobic conditions; tests of gas production from D-glucose and glucose; tests of acid production from amygdalin, L-arabinose, cellobiose, D-fructose, D-galactose, D-glucose, lactose, maltose, D-mannitol, D-mannose, melezitose, melibiose, rhamnose, L-rhamnose, D-ribose, salicin, D-sorbitol, sucrose, trehalose and D-xylose; and growth tests in terms of esculin hydrolysis and L-arginine deamination. The positive or negative result of each test is inputted into the Micro-IS computer identification program to yield the taxonomic genus or species of the identified bacteria.

(4) 16S rDNA sequence analysis:

This identification test is based on the method described in Rosenblum et al. (1997), Nucleic Acid Res., 25: 4500-4504, and it works according to the following operating procedures: A small amount of a freshly cultured bacterial strain is scraped and placed into a 1.5 ml eppendorf tube. After treating the bacterial sample with proteinase K and RNase and using a spin column to obtain the bacterial genomic DNA therefrom, PCR amplification is conducted, followed by purification. After confirmation by electrophoresis, the PCR-amplified products are subjected to reaction with a MicroSeq™ 16SrDNA Gene Kit (PE Co., USA), followed by DNA sequencing using the ABI Prism 310 Genetic Analyzer. Furthermore, the 12 strips of DNA sequences thus obtained are analyzed and integrated by using MicroSeq™ software (PE Co., USA) to obtain a 16S rDNA sequence of about 1500 bps, said sequence being subjected to a comparison with DNA databases (MicroSeq™ Reference Manual, 1999, PE CO., USA).

II. Results:

1. **Isolate B21T1 of the present invention**

(i) According to the results of preliminary identification test, the isolate B21T1 is gram-positive, catalase negative and non-mobile, and it grows under both aerobic and anaerobic conditions;
(ii) The test results using API 50 CHL identification kit of API System are shown in Table 6, in which the identification score is 91.0 (%ID). The identity of this isolate is confirmed to be *Lactobacillus acidophilus;*
(iii) The isolate B21T1 of the present invention is identified as *Lactobacillus acidophilus* by the Micro-IS System, in which the obtained results are shown in Table 7 and the identification score is 0.682 (ID score);
(iv) The 16S rDNA sequence analysis result of the isolate B21T1 of the present invention is shown in Figure 5. After a comparison with DNA databases (MicroSeq™ Reference Manual, 1999, PE CO., USA), it was found that the 16S rDNA sequence (SEQ ID NO:1) of the isolate B21T1 of the present invention is most homologous to the 16S rDNA sequence of *Lactobacillus gasseri* ($B_1$ group in *Lactobacillus acidophilus*)(G. Klein et al. (1998), International Journal of Food Microbiology. 41:103-125); and

(v) According to the above-mentioned identification results, the isolate B21T1 of the present invention is most probably *Lactobacillus gasseri*, subgroup $B_1$ of *Lactobacillus acidophilus.*

Table 6

Reference: B21T1

DOUBTFUL PROFILE

---

Strip : API 50 CHL

Profile : – – – – – – – – – – – – + – + ? – – – – – – – + + – + + + + – – + – – – – – – – – + – – – – – – – – – – –

| | | | | | | | | | | | | | | | | | | | | | |
|---|---|---|---|---|---|---|---|---|---|---|---|---|---|---|---|---|---|---|---|---|---|
| 0 | – | GLY | – | ERY | – | DARA | – | LARL | – | RIB | – | DXYL | – | LXYL | – | ADO | – | MDX | – | GAL | – |
| GLU | + | FRU | – | MNE | + | SBE | ? | RHA | – | DUL | – | INO | – | MAN | – | SOR | – | MDM | – | MDG | – |
| NAG | + | AMY | + | ARB | – | ESC | + | SAL | + | CEL | + | MAL | + | LAC | – | MEL | – | SAC | + | TRE | – |
| INU | – | MLZ | – | RAF | – | AMD | – | GLYG | – | XLT | – | GEN | + | TUR | – | LYX | – | TAG | – | DFUC | – |
| LFUC | – | DARL | – | LARL | – | GNT | – | 2KG | – | 5KG | – | | | | | | | | | | |

| Significant taxa | | % Id. | T | Tests against |
|---|---|---|---|---|
| *Lacto. acidophilus* | 1 | 91.0 | 0.53 | 3 |
| Next choice | | | | |
| *Lacto. crispatus* | 2 | 5.4 | 0.35 | 4 |

---

*Lacto. acidophilus* 1 : 3 test(s) against

| | | | | | |
|---|---|---|---|---|---|
| GALACTOSE | (GAL) | 75 % | D-FRUCTOSE | (FRU) | 100 % |
| TREHALOSE | (TRE) | 77 % | | | |

Table 7

**Micro-IS System**

Strain names: **B21T1, B21T6, C21T1, X21B7, B6T7**

| | |
|---|---|
| - 013001 MOTILITY | - 006001 ENDOSPORES |
| - 017013 GROWTH 15°C | + 017017 GROWTH 45 °C |
| + 016059 AEROBIC GROWTH | - 024045 GLUCOSE TO $CO_2$ |
| - 028528 GLUCONATE, GAS | + 025203 AMYGDALIN, ACID |
| - 025181 L-ARABINOSE, ACID | + 025211 CELLOBIOSE, ACID |
| + 025193 D-FRUCTOSE, ACID | + 025194 D-GALACTOSE, ACID |
| + 025195 D-GLUCOSE, ACID | + 025212 LACTOSE, ACID |
| + 025213 MALTOSE, ACID | - 026371 D-MANNITOL, ACID |
| + 025196 D-MANNOSE, ACID | - 025217 MELEZITOSE, ACID |
| + 025214 MELIBIOSE, ACID | + 025218 RAFFINOSE, ACID |
| - 025191 L-RHAMNOSE, ACID | - 025184 D-RIBOSE, ACID |
| + 025210 SALICIN, ACID | - 026374 D-SORBITOL, ACID |
| + 025215 SUCROSE, ACID | + 025216 TREHALOSE, ACID |
| - 025186 D-XYLOSE, ACID | + 028060 L-MALATE UTIL |

| ***DIAGNOSIS*** | ****ID SCORE** | ****LIEKLIHOOD** | ****BESTLIKEL.** |
|---|---|---|---|
| 1 *L. acidophilus* | 0.68224 | 0.079110108 | 0.0791101 |
| 2 *L. vitulinus* | 0.31761 | 0.036828671 | 0.1104860 |

| **SUGGESTED TESTS** | ****VALUE IN SET**VALUE ALONE** |
|---|---|
| 1 024029 (L+) LACTATE PROD 1 | |

**2. Isolate B21T6 of the present invention**

(i) According to the preliminary identification results, the isolate B21T6 is gram-positive, catalase negative and non-mobile, and it grows in both aerobic and anaerobic conditions;

(ii) The test results using API 50 CHL identification kit of the API System are shown in Table 8, in which the identification score is 93.6 (%ID). The identity of this isolate is confirmed to be *Lactobacillus acidophilus;*

(iii) The isolate B21T6 of the present invention is identified to be *Lactobacillus acidophilus* by the Micro-IS System, in which the obtained results are shown in Table 7, and the identification score is 0.682 (ID score);

(iv) The 16S rDNA sequence analysis result of the isolate B21T6 of the present invention is shown in Figure 6. After a comparison with DNA databases (MicroSeq™ Reference Manual, 1999, PE CO., USA), it was found that the 16S rDNA sequence (SEQ ID NO:2) of the isolate B21T6 of the present invention is most homologous to the 16S rDNA sequence of *Lactobacillus gasseri* ($B_1$ group in *Lactobacillus acidophilus);* and

(v) According to the above-mentioned identification results, the isolate B21T6 of the present invention is most probably *Lactobacillus gasseri*, subgroup $B_1$ of *Lactobacillus acidophilus.*

Table 8

Reference: B21T6

VERY GOOD IDENTIFICATION TO THE GENUS

Strip : API 50 CHL

Profile : ---------- -++------- --+--+++-- -+-------- ----------

| | | | | | | | | | | | | | | | | | | | | | |
|---|---|---|---|---|---|---|---|---|---|---|---|---|---|---|---|---|---|---|---|---|---|
| 0 | - | GLY | - | ERY | - | DARA | - | LARL | - | RIB | - | DXYL | - | LXYL | - | ADO | - | MDX | - | GAL | - |
| GLU | + | FRU | + | MNE | - | SBE | - | RHA | - | DUL | - | INO | - | MAN | - | SOR | - | MDM | - | MDG | - |
| NAG | + | AMY | + | ARB | - | ESC | + | SAL | + | CEL | + | MAL | + | LAC | - | MEL | - | SAC | + | TRE | - |
| INU | - | MLZ | - | RAF | - | AMD | - | GLYG | - | XLT | - | GEN | + | TUR | - | LYX | - | TAG | - | DFUC | - |
| LFUC | - | DARL | - | LARL | - | GNT | - | 2KG | - | 5KG | - | | | | | | | | | | |

| Significant taxa | | % Id. | T | Tests against |
|---|---|---|---|---|
| *Lacto. acidophilus* | 3 | 93.6 | 0.80 | 1 |
| *Lacto. delb.delb.* | | 6.0 | 0.65 | 5 |
| Next choice | | | | |
| *Lacto. crispatus* | | 0.2 | 0.39 | 4 |

*Lacto. acidophilus* 1 : 1 test(s) against

D-MALTOSE (MAL) 75 %

**3. Isolate C21T1 of the present invention**

(i) According to the preliminary identification results, the isolate C21T1 is gram-positive, catalase negative and non-mobile, and it grows under aerobic and anaerobic conditions;

(ii) The test results using API 50 CHL identification kit of the API System are shown in Table 9, in which the identification score is 95.6 (%ID). The identity of this isolate is confirmed to be *Lactobacillus acidophilus;*

(iii) The isolate C21T1 of the present invention is identified to be *Lactobacillus acidophilus* by the Micro-IS System, in which the obtained results are shown in Table 7, and the identification score is 0.682 (ID score);

(iv) The 16S rDNA sequence analysis result of the isolate C21T1 of the present invention is shown in Figure 7. After a comparison with DNA databases (MicroSeq™ Reference Manual, 1999, PE CO., USA), it was found that the 16S rDNA sequence (SEQ ID NO:3) of the isolate C21T1 of the present invention is most homologous to the 16S rDNA sequence of *Lactobacillus gasseri* ($B_1$ group in *Lactobacillus acidophilus);* and

(v) According to the above-mentioned identification results, the isolate C21T1 of the present invention is most probably *Lactobacillus gasseri*, subgroup $B_1$ of *Lactobacillus acidophilus.*

Table 9

Reference: C21T1
GOOD IDENTIFICATION

Strip : API 50 CHL
Profile : ---------- -++------- --++-++++- -+-------+ ----------

| | | | | | | | | | | | | | | | | | | | | | |
|---|---|---|---|---|---|---|---|---|---|---|---|---|---|---|---|---|---|---|---|---|---|
| 0 | - | GLY | - | ERY | - | DARA | - | LARL | - | RIB | - | DXYL | - | LXYL | - | ADO | - | MDX | - | GAL | - |
| GLU | + | FRU | + | MNE | - | SBE | - | RHA | - | DUL | - | INO | - | MAN | - | SOR | - | MDM | - | MDG | - |
| NAG | + | AMY | + | ARB | - | ESC | + | SAL | + | CEL | + | MAL | + | LAC | - | MEL | - | SAC | + | TRE | - |
| INU | - | MLZ | - | RAF | - | AMD | - | GLYG | - | XLT | - | GEN | + | TUR | - | LYX | - | TAG | - | DFUC | - |
| LFUC | - | DARL | - | LARL | - | GNT | - | 2KG | - | 5KG | - | | | | | | | | | | |

| Significant taxa | | % Id. | T | Tests against |
|---|---|---|---|---|
| *Lacto. acidophilus* | 1 | 95.6 | 0.70 | 3 |
| Next choice | | | | |
| *Lacto. crispatus* | | 1.5 | 0.53 | 5 |

*Lacto. acidophilus* 1 : 3 test(s) against

| | | | | | |
|---|---|---|---|---|---|
| GALACTOSE | (GAL) | 75 % | D-MANNOSE | (MNE) | 96 % |
| TREHALOSE | (TRE) | 77 % | | | |

**4. Isolate X21B7 of the present invention**

(i) According to the preliminary identification results, the isolate X21B7 is gram-positive, catalase negative and non-mobile, and it grows under both aerobic and anaerobic conditions;
(ii) The test results using API 50 CHL identification kit of the API System are shown in Table 10, in which the identification score is 94.3 (%ID). The identity of this isolate is confirmed to be *Lactobacillus acidophilus;*
(iii) The isolate X21 B7 of the present invention is identified to be *Lactobacillus acidophilus* by the Micro-IS System, in which the obtained results are shown in Table 7, and the identification score is 0.682 (ID score);
(iv) The 16S rDNA sequence analysis result of the isolate X21B7 of the present invention is shown in Figure 8. After a comparison with DNA databases (MicroSeq™ Reference Manual, 1999, PE CO., USA), it was found that the 16S rDNA sequence (SEQ ID NO:4) of the isolate X21 B7 of the present invention is most homologous to the 16S rDNA sequence of *Lactobacillus gasseri* ($B_1$ group in *Lactobacillus acidophilus);* and
(v) According to the above-mentioned identification results, the isolate X21B7 of the present invention is most probably *Lactobacillus gasseri*, subgroup $B_1$ of *Lactobacillus acidophilus.*

Table 10

Reference: X21B7
GOOD IDENTIFICATION

Strip : API 50 CHL
Profile : – – – – – – – – – – ++ ? – – – – – – – – –++–++++– –+– – – – – – –+ – – – – – – – – – –

| | | | | | | | | | | | | | | | | | | | |
|---|---|---|---|---|---|---|---|---|---|---|---|---|---|---|---|---|---|---|---|
| 0 | – | GLY | – | ERY | – | DARA | – | LARL | – | RIB | – | DXYL | – | LXYL | – | ADO | – | MDX | – | GAL | + |
| GLU | + | FRU | ? | MNE | – | SBE | – | RHA | – | DUL | – | INO | – | MAN | – | SOR | – | MDM | – | MDG | – |
| NAG | + | AMY | + | ARB | – | ESC | + | SAL | + | CEL | + | MAL | + | LAC | – | MEL | – | SAC | + | TRE | – |
| INU | – | MLZ | – | RAF | – | AMD | – | GLYG | – | XLT | – | GEN | + | TUR | – | LYX | – | TAG | – | DFUC | – |
| LFUC | – | DARL | – | LARL | – | GNT | – | 2KG | – | 5KG | – | | | | | | | | | | |

| Significant taxa | | % Id. | T | Tests against |
|---|---|---|---|---|
| *Lacto. Acidophilus* | 1 | 94.3 | 0.65 | 2 |
| Next choice | | | | |
| *Pedio. Damnosus* | 2 | 2.0 | 0.43 | 3 |

*Lacto. acidophilus* 1 : 2 test(s) against
D-MANNOSE (MNE) 96 % TREHALOSE (TRE) 77 %

5. **Isolate B38T38 of the present invention**

(i) According to the preliminary identification results, the isolate B38T38 is gram-positive, catalase negative and non-mobile, and it grows under both aerobic and anaerobic conditions; (ii) The test results using API 50 CHL identification kit of the API System are shown in Table 11, in which the identification score is 90.8 (%ID). The identity of this isolate is confirmed to be *Lactobacillus acidophilus;*
(iii) The isolate B38T38 of the present invention is identified to be *Lactobacillus acidophilus* by the Micro-IS System, in which the obtained results are shown in Table 12, and the identification score is 0.646 (ID score);
(iv) The 16S rDNA sequence analysis result of the isolate B38T38 of the present invention is shown in Figure 9. After a comparison with DNA databases (MicroSeq™ Reference Manual, 1999, PE CO., USA), it was found that the 16S rDNA sequence (SEQ ID NO:5) of the isolate B38T38 of the present invention is most homologous to the 16S rDNA sequence of *Lactobacillus gasseri* ($B_1$ group in *Lactobacillus acidophilus*); and
(v) According to the above-mentioned identification results, the isolate B38T38 of the present invention is most probably *Lactobacillus gasseri*, subgroup $B_1$ of *Lactobacillus acidophilus*

Table 11

Reference: B38T38
GOOD IDENTIFICATION

---

Strip : API 50 CHL
Profile : ---------- ++++------ --+-+++++- -++------+ ----------

| | | | | | | | | | | | | | | | | | | | | | |
|---|---|---|---|---|---|---|---|---|---|---|---|---|---|---|---|---|---|---|---|---|---|
| 0 | – | GLY | – | ERY | – | DARA | – | LARL | – | RIB | – | DXYL | – | LXYL | – | ADO | – | MDX | – | GAL | + |
| GLU | + | FRU | + | MNE | + | SBE | – | RHA | – | DUL | – | INO | – | MAN | – | SOR | – | MDM | – | MDG | – |
| NAG | + | AMY | – | ARB | + | ESC | + | SAL | + | CEL | + | MAL | + | LAC | – | MEL | – | SAC | + | TRE | + |
| INU | – | MLZ | – | RAF | – | AMD | – | GLYG | – | XLT | – | GEN | + | TUR | – | LYX | – | TAG | – | DFUC | – |
| LFUC | – | DARL | – | LARL | – | GNT | – | 2KG | – | 5KG | – | | | | | | | | | | |

| Significant taxa | | % Id. | T | Tests against |
|---|---|---|---|---|
| *Lacto. acidophilus* | 1 | 90.8 | 0.93 | 0 |
| Next choice | | | | |
| *Lacto. delb.lactis* | 2 | 6.2 | 0.74 | 2 |

*Lacto. acidophilus* 1 : 0 test(s) against

Table 12

**Micro-IS System**
Strain name: **B38T38**

| | | | |
|---|---|---|---|
| - 013001 MOTILITY | | - 006001 | ENDOSPORES |
| - 017013 GROWTH 15°C | | + 017017 | GROWTH 45 °C |
| + 016059 AEROBIC GROWTH | | - 024045 | GLUCOSE TO $CO_2$ |
| - 028528 GLUCONATE, GAS | | + 025203 | AMYGDALIN, ACID |
| - 025181 L-ARABINOSE, ACID | | + 025211 | CELLOBIOSE, ACID |
| + 025193 D-FRUCTOSE, ACID | | + 025194 | D-GALACTOSE, ACID |
| + 025195 D-GLUCOSE, ACID | | + 025212 | LACTOSE, ACID |
| + 025213 MALTOSE, ACID | | - 026371 | D-MANNITOL, ACID |
| + 025196 D-MANNOSE, ACID | | + 025217 | MELEZITOSE, ACID |
| + 025214 MELIBIOSE, ACID | | + 025218 | RAFFINOSE, ACID |
| - 025191 L-RHAMNOSE, ACID | | - 025184 | D-RIBOSE, ACID |
| + 025210 SALICIN, ACID | | - 026374 | D-SORBITOL, ACID |
| + 025215 SUCROSE, ACID | | + 025216 | TREHALOSE, ACID |
| - 025186 D-XYLOSE, ACID | | - 028060 | L-MALATE UTIL |
| ***DIAGNOSIS*** | ****ID SCORE** | ****LIKELIHOOD** | ****BESTLIKEL.** |
| *1 L. delbrueckll ssp lactis* | 0.64607 | 0.145038024 | 0.4351140 |
| *2 L. acidophilus* | 0.35240 | 0.079110108 | 0.0791101 |
| **SUGGESTED TE** | | ****VALUE IN SET** | ****VALUE ALONE** |
| 1 024029 (L+) LACTATE PROD | | | 2 |
| 2 029268 L-ARGININE DEAM | | | 2 |

**6. Isolate B6T7 of the present invention**

(i) According to the preliminary identification results, the isolate B6T7 is gram-positive, catalase negative and non-mobile, and it grows under both aerobic and anaerobic conditions;

(ii) The test results using API 50 CHL identification kit of the API System are shown in Table 13, in which the identification score is 92.4 (%ID). The identity of this isolate is confirmed to be *Lactobacillus plantarum*;

(iii) The isolate B6T7 of the present invention is identified to be *Lactobacillus acidophilus* by the Micro-IS System, in which the obtained results are shown in Table 7, and the identification score is 0.682 (ID score);

(iv) The 16S rDNA sequence analysis result of the isolate B6T7 of the present invention is shown in Figure 10. Since the identification results obtained with the API 50 CHL identification kit and those obtained with the Micro-IS System identification system classify the isolate B6T7 of the present invention to be *Lactobacillus plantarum* and *Lactobacillus acidophilus* of *Lactobacillus,* respectively, the 16S rDNA sequence (SEQ ID NO: 6) of the isolate B6T7 of the present invention was compared with the 16S rDNA sequence of *Lactobacillus acidophilus* (SEQ ID NO:7) and that of *Lactobacillus plantarum* (SEQ ID NO:8). It was found that the 16S rDNA sequence of the isolate B6T7 (SEQ ID NO:6) of the present invention is most homologous to the 16S rDNA sequence of *Lactobacillus acidophilus,* and differs greatly from the 16S rDNA sequence of *Lactobacillus plantarum*; and

(v) According to the above identification results, the isolate B6T7 of the present invention is most probably *Lactobacillus acidophilus.*

Table 13

Reference: B6T7

DOUBTFUL PROFILE

Strip : API 50 CHL

Profile : −−−−++−−−− ++++−+−−+− −−+++−++++ +++−−+−−−+ −−−−−−−−−−

| | | | | | | | | | | | | | | | | | | | | | | | |
|---|---|---|---|---|---|---|---|---|---|---|---|---|---|---|---|---|---|---|---|---|---|---|---|
| 0 | − | GLY | − | ERY | − | DARA | − | LARL | + | RIB | + | DXYL | − | LXYL | − | ADO | − | MDX | − | GAL | + | | |
| GLU | + | FRU | + | MNE | + | SBE | − | RHA | + | DUL | − | INO | − | MAN | + | SOR | − | MDM | − | MDG | − | | |
| NAG | + | AMY | + | ARB | + | ESC | − | SAL | + | CEL | + | MAL | + | LAC | + | MEL | + | SAC | + | TRE | + | | |
| INU | − | MLZ | − | RAF | + | AMD | − | GLYG | − | XLT | − | GEN | + | TUR | − | LYX | − | TAG | − | DFUC | − | | |
| LFUC | − | DARL | − | LARL | − | GNT | − | 2KG | − | 5KG | − | | | | | | | | | | | | |

| Significant taxa | | % Id. | T | Tests against |
|---|---|---|---|---|
| *Lacto. plantarum* | 1 | 92.4 | 0.59 | 3 |
| Next choice | | | | |
| *Lacto. salivarius* | | 3.4 | 0.45 | 4 |

*Lacto. plantarum* 1 : 3 test(s) against

| | | | | | |
|---|---|---|---|---|---|
| D-SORBITOL | (SPR) | 75 % | ESCULINE | (ESC) | 99 % |
| MELEZITOSE | (MLE) | 92 % | | | |

Summarizing the identification results, all of the six isolates screened in accordance with the present invention belong to *Lactobacillus* strains, in which B6T7 is *Lactobacillus acidophilus,* and B21T1, B21T6, C21T1, X21B7 and B38T38 are *Lactobacillus gasseri*, subgroup $B_1$ of *Lactobacillus acidophilus.*

Referring to Table 14, as compared with *Lactobacillus* strains disclosed in prior patents and literatures, the six *Lactobacillus* isolates obtained in accordance with the present invention not only can grow in an environment containing 0.3% bile salts, but also have higher survival rates and a good cholesterol-lowering ability after being cultured in an acidic environment of pH 2 for 2 hours. In addition, the six *Lactobacillus* isolates of the present invention have the ability to co-precipitate with cholesterol and assimilate cholesterol. It is apparent that the isolates of the present invention and their sub-cultured offsprings are excellent probiotic bacteria, and can be used in the manufacture of food products, such as beverages, cakes, infant foods, fermented milk, dietary supplements, and animal feed. Also, they can be used in the manufacture of pharmaceutical compositions for the treatment and prevention of gastrointestinal diseases and lowering serum cholesterol.

For examples, *Lactobacillus* isolates obtained in accordance with the present invention can be used in the manufacture of lactic acid beverages and yogurt drinks with reference to Referential Example 1 and Referential Example 2 disclosed in USP 5,516,684.

Table 14. Comparison of the isolates of the present invention with known strains disclosed in prior patents and literatures

| Strains | Sources of isolation | Characteristics | patent/literature |
|---|---|---|---|
| *L. acidophilus*<br>GG (ATCC 53103) | Human | 1.Bile salt tolerance<br>It can grow In an environment containing 0.15% bile acids.<br>2.Acld tolerance It can grow up to 10° CFU in an pH 5 environment.<br>It can grow up to 10’ CFU in an pH 3 environment.<br>Population decreased within 2 log values after being grown in pH 2.5 gastric juice for 30 minutes.<br>Survived cells after treatment at pH 1-2 for 2 hrs were measured to be $10^3$ CFU.<br>3.Lowering of cholesterol<br>It lowered the excretion amount of cholesterol in human feces. | US 4839281<br>US 5032399 |

(continued)

| Strains | Sources of isolation | Characteristics | patent/literature |
|---|---|---|---|
| *L. acidophilus* LA16 | Male pig | 1.Lowerlng of cholesterol<br>A decrease of serum cholesterol by 60% (in a system containing 0.4% oxgall and 10 mg cholesterol).<br>Animal tests revealed that after a feeding time of 56 days, serum cholesterol was decreased by 10.5% and LDL-cholesterol content was decreased by 9%.<br>(Note : LA16 is a *L. acidophilus* strain Isolated from the feces of pigs which had been fed with yogurt containing *L. acidophilus* DDS-1 (human source) and having the ability of lowering cholesterol.) | Danielson *et al.,* 1989 |
| *L. acidophilus*<br>ATCC 43121 | Pig | 1.Bile salt tolerance<br>The $OD_{620nm}$ values of the two strains were Increased by 0.3 units after being cultured in an environment containing 0.3% oxgall for 2.93 and 7.4 hours, respectively. | Gilliland and Walker, 1990 |
| ATCC 4356 | Human | 2.Lowering of cholesterol<br>A decrease of serum cholesterol by 55.4% and 12.9%, respectively, after being cultured in a system containing 0.3% oxgall and 0.1% PPLO for 14 hrs. | |

(continued)

| Strains | Sources of isolation | Characteristics | patent/literature |
|---|---|---|---|
| *L. gasseri*<br>SBT 0274<br>SBT 0270 | Human | 1.BIle salt tolerances The two strains can grow in an environment containing 0.3% oxgall.<br>2.Acid tolerance Population decreased 2 log values after being grown at pH 2.5, 37°C for 3 hrs. Population decreased 4-5 log values after being grown at pH 1.5, 37°C for 2 hrs<br>3.Lowering of cholesterol A decrease of cholesterol content by 47 % and 36 %, respectively, after being cultured in a system containing 0.3% oxgall and 100 μg cholesterol mlcelle/ml for 20 hrs. | Usman and Hosono, 1999 |
| *L. gasseri*<br>B21T1<br>B21T6<br>C21T1<br>X21B7<br>B38T38 | Healthy infants | 1.Bile salt tolerance 80% survived, *i.e.* population decreased 0.5-2 log values after being grown in an environment containing 0.3% oxgall for 24 hrs.<br>2.Acid tolerance Population decreased 3 ~ 4 log values after being treated at pH 2, 37°C for 2 hrs (0.85% NaCl/ 0.01 N HCl system) | The invention strains |

(continued)

| Strains | Sources of isolation | Characteristics | patent/literature |
|---|---|---|---|
| *L. acidophilus* B6T7 | | 3.Lowering of cholesterol A decrease of cholesterol content by 93% or higher after anaerobic culture in MRS broth containing 0.3% oxgall and 12% horse serum for 24 hrs. A decrease of cholesterol content by 25~45% after anaerobic culture in MRS broth containing 0.3% oxgall and 12% cholesterol micelles for 24 hrs. A cholesterol assimilation rate reached 12-40%. | |
| Note : ATCC 43121= CCRC 17064 ATCC 4356 = CCRC 10695 PPLO (BACT PPLO SERUM FRACTION) was produced by DIFCO LABORATORIES, but this product has not been produced presently. | | | |

## Claims

**1.** An isolated strain of *Lactobacillus sp.,* wherein the strain is:

(i) a deposited strain selected from:

(a) *Lactobacillus gasseri* B21T1 deposited in the American Type Culture Collection under accession number ATCC PTA-4483;
(b) *Lactobacillus gasseri* B21T6 deposited in the ATCC under accession number ATCC PTA-4484;
(c) *Lactobacillus gasseri* C21T1 deposited in the ATCC under accession number ATCC PTA-4479;
(d) *Lactobacillus gasseri* X21B7 deposited in the ATCC under accession number ATCC PTA-4480;
(e) *Lactobacillus gasseri* B38T38 deposited in the ATCC under accession number ATCC PTA-4481; or
(f) *Lactobacillus acidophilus* B6T7 deposited in the ATCC under accession number ATCC PTA-4482;

(ii) the sub-cultured offspring of the deposited strain (i); or
(iii) a mutant derived from the deposited strain (i);

wherein the sub-cultured offspring (ii) or mutant (iii) exhibits the bacteriological characteristics of:-

a population decrease of about 0.5 to 2 log values after growth in an environment containing 0.3% oxgall for 24 hours;
an acid tolerance as exhibited by a 3 to 4 log value population decrease after treatment at pH 2 at 37°C for 2 hours, in a 0.85% NaCl/0.01N HCl system;
the ability to decrease cholesterol content by 93% or higher after anaerobic culture in broth containing 0.3% oxgall and 12% horse serum for 24 hours;
the ability to decrease cholesterol content by 25 to 45% after anaerobic culture in broth containing 0.3% oxgall and 12% cholesterol micelles for 24 hours; and
a cholesterol assimilation rate of 12 to 40%.

**2.** A food product comprising an edible material and an isolated strain of *Lactobacillus sp.* according to claim 1.

**3.** The food product of claim 2, further comprising at least a probiotic microorganism selected from the group consisting of: *Lactobacillus sp.,* such as *Lactobacillus acidophilus, Lactobacillus lactis, Lactobacillus brevis, Lactobacillus casei, Lactobacillus plantarum, Lactobacillus salivarius, Lactobacillus bifidus, Lactobacillus bulgaricus, Lactobacillus causasicus* and *Lactobacillus rhamnosus; Streptococcus sp.,* such as *Streptococcus thermophilus,* and *Strep-*

*tococcus lactis;* yeasts, such as *Candida Kefyr*, and *Saccharomyces florentinus*; or a combination thereof.

**4.** The food product of claim 2 or claim 3, wherein the edible material is selected from the group consisting of: fluid milk products, fermented milk, milk powder, ice cream, cream cheese, dry cheese, bean milk and fermented bean milk, fruit-vegetable juices, fruit juices, sports drinks, dessert, candy, infant formulas, health food products, animal feed and dietary supplements.

**5.** The food product of any of claims 2 to 4, which is manufactured in the form of an instant food.

**6.** A pharmaceutical composition comprising a probiotically effective amount of an isolated strain of *Lactobacillus sp.* according to claim 1.

**7.** The pharmaceutical composition of claim 6, wherein the composition is formulated into an oral dosage form selected from the group consisting of solution, emulsion, powder, tablet and capsule.

**8.** The pharmaceutical composition of claim 6 or claim 7, wherein the composition is manufactured as a digestion improving drug.

**9.** The pharmaceutical composition of claim 6 or claim 7, wherein the composition is manufactured as a drug for lowering serum cholesterol.

**10.** The use of a strain of *Lactobacillus sp.* of claim 1 for the manufacture of a medicament for the treatment or prevention of gastrointestinal diseases or for lowering serum cholesterol.

**Patentansprüche**

**1.** Isolierter Stamm von *Lactobacillus sp.,* wobei der Stamm:

(i) ein hinterlegter Stamm, gewählt aus:

(a) *Lactobacillus gasseri* B21T1, hinterlegt in der Amerikanischen Kultur-Typ-Sammlung (American Type Culture Collection) unter der Zugangsnummer ATCC PTA-4483;
(b) *Lactobacillus gasseri* B21T6, hinterlegt bei der ATCC unter der Zugangsnummer ATCC PTA-4484;
(c) *Lactobacillus gasseri* C21T1, hinterlegt bei der ATCC unter der Zugangsnummer ATCC PTA-4479;
(d) *Lactobacillus gasseri* X21 B7, hinterlegt bei der ATCC unter der Zugangsnummer ATCC PTA-4480;
(e) *Lactobacillus gasseri* B38T38, hinterlegt bei der ATCC unter der Zugangsnummer ATCC PTA-4481; oder
(f) *Lactobacillus acidophilus* B6T7, hinterlegt bei der ATCC unter der Zugangsnummer ATCC PTA-4482;

(ii) der subkultivierte Abkömmling des hinterlegten Stamms (i); oder
(iii) ein von dem hinterlegten Stamm (i) abgeleitete Mutante ist;

wobei der/die subkultivierte Abkömmling (ii) oder Mutante (iii) die folgenden bakteriologischen Charakteristika zeigt:

eine Populationsabnahme von etwa 0,5 bis 2 log-Werten nach dem Wachstum in einer 0,3 % Ochsengalle enthaltenden Umgebung während 24 Stunden;
eine Säuretoleranz, wie durch eine Populationsabnahme mit einem 3- bis 4-log-Wert nach der Behandlung bei einem pH-Wert von 2 bei 37°C während 2 Stunden in einem 0,85 % NaCl/0,01 N HCl-System;
die Fähigkeit zur Senkung des Cholesterolgehalts um 93 % oder mehr nach der anaeroben Kultivierung in Kulturbrühe, enthaltend 0,3 % Ochsengalle und 12 % Pferdeserum, während 24 Stunden; und
eine Cholesterol-Assimilationsrate von 12 bis 40 %.

**2.** Lebensmittelprodukt, umfassend ein essbares Material und einen isolierten Stamm von *Lactobacillus sp.* gemäß Anspruch 1.

**3.** Lebensmittelprodukt gemäß Anspruch 2, weiterhin umfassend mindestens einen probiotischen Mikroorganismus, gewählt aus der Gruppe bestehend aus: *Lactobacillus sp.,* wie *Lactobacillus acidophilus, Lactobacillus lactis, Lactobacillus brevis, Lactobacillus casei, Lactobacillus plantarum, Lactobacillus salivarius, Lactobacillus bifidus, Lac-*

*tobacillus bulgaricus, Lactobacillus causasicus* und *Lactobacillus rhamnosus; Streptococcus sp.,* wie *Streptococcus thermophilus* und *Streptococcus lactis;* Hefen, wie *Candida Kefyr,* und *Saccharomyces florentinus;* oder eine Kombination hiervon.

**4.** Lebensmittelprodukt gemäß Anspruch 2 oder Anspruch 3, wobei das essbare Material gewählt ist aus der Gruppe bestehend aus: flüssigen Milchprodukten, fermentierter Milch, Milchpulver, Eiscreme, Rahmkäse, Trockenkäse, Bohnenmilch und fermentierter Bohnenmilch, Frucht-Gemüse-Säften, Fruchtsäften, Sportgetränken, Dessert, Kandiszucker, Kinder(fertig)nahrungen, Gesundheits-Nahrungsmittelprodukten, Tierfutter und dietätischen Ergänzungen.

**5.** Lebensmittelprodukt gemäß mindestens einem der Ansprüche 2 bis 4, das in der Form einer Fertignahrung hergestellt wird.

**6.** Pharmazeutische Zusammensetzung, umfassend eine probiotisch wirksame Menge eines isolierten Stamms von *Lactobacillus sp.* gemäß Anspruch 1.

**7.** Pharmazeutische Zusammensetzung gemäß Anspruch 6, wobei die Zusammensetzung zu einer oralen Dosierungsform formuliert ist, gewählt aus der Gruppe bestehend aus Lösung, Emulsion, Pulver, Tablette und Kapsel.

**8.** Pharmazeutische Zusammensetzung gemäß Anspruch 6 oder Anspruch 7, wobei die Zusammensetzung als ein die Verdauung verbessernder Arzneistoff hergestellt ist.

**9.** Pharmazeutische Zusammensetzung gemäß Anspruch 6 oder Anspruch 7, wobei die Zusammensetzung als ein Arzneistoff zur Senkung von Serumcholesterol hergestellt wird.

**10.** Verwendung eines Stamms von *Lactobacillus sp.* gemäß Anspruch 1 für die Herstellung eines Medikaments für die Behandlung oder Prävention von gastrointestinalen Erkrankungen oder für die Senkung von Serumcholesterol.

## Revendications

**1.** Souche isolée de *Lactobacillus sp.,* où la souche est :

(i) une souche déposée choisie parmi :

(a) *Lactobacillus gasseri* B21T1 déposée à l'American Type Culture Collection sous le numéro d'accès ATCC PTA-4483
(b) *Lactobacillus gasseri* B21T6 déposée à l'ATCC sous le numéro d'accès ATCC PTA-4484
(c) *Lactobacillus gasseri* C21T1 déposée à l'ATCC sous le numéro d'accès ATCC PTA-4479
(d) *Lactobacillus gasseri* X21B7 déposée à l'ATCC sous le numéro d'accès ATCC PTA-4480
(e) *Lactobacillus gasseri* B38T38 déposée à l'ATCC sous le numéro d'accès ATCC PTA-4481 ; ou
(f) *Lactobacillus acidophilus* B6T7 déposée à l'ATCC sous le numéro d'accès ATCC PTA-4482

(ii) la descendance sous-cultivée d'une souche déposée (i) ; ou
(iii) un mutant dérivé de la souche déposée (i) ;

où la descendance sous-cultivée (ii) ou le mutant (iii) présente les caractéristiques bactériologiques de :

une diminution de la population d'environ 0,5 à 2 valeurs de log après la croissance dans un environnement contenant 0,3 % d'oxgall durant 24 heures ;
une tolérance à l'acide telle qu'elle se présente par une diminution de la population de 3 à 4 valeurs de log après le traitement à pH 2 à 37°C durant 2 heures, dans un système de NaCl à 0,85 %/ HCl 0,01 N ;
la capacité à diminuer la teneur en cholestérol de 93 % ou plus après la culture anaérobie dans du milieu contenant 0,3 % d'oxgall et 12 % de sérum de cheval durant 24 heures ;
la capacité à abaisser la teneur en cholestérol de 25 à 45 % après la culture anaérobie dans du milieu contenant 0,3 % d'oxgall et 12 % de micelles de cholestérol durant 24 heures ; et
un taux d'assimilation du cholestérol de 12 à 40 %.

2. Produit alimentaire comprenant une matière comestible et une souche isolée de *Lactobacillus sp.* selon la revendication 1.

3. Produit alimentaire selon la revendication 2, comprenant en outre au moins un microorganisme probiotique choisi dans le groupe constitué par : *Lactobacillus sp.,* tel que *Lactobacillus acidophilus, Lactobacillus lactis, Lactobacillus brevis, Lactobacillus casei, Lactobacillus plantarum, Lactobacillus salivarius, Lactobacillus bifidus, Lactobacillus bulgaricus, Lactobacillus causasicus et Lactobacillus rhamnosus ; Streptococcus sp.,* tel que *Streptococcus thermophilus* et *Streptococcus lactis ;* les levures, telles que *Candida kefyr* et *Saccharomyces florentinus ;* ou une combinaison de ceux-ci.

4. Produit alimentaire selon la revendication 2 ou la revendication 3, où la matière comestible est choisie dans le groupe constitué par : les produits laitiers fluides, le lait fermenté, la poudre de lait, la crème glacée, le fromage frais à tartiner, le fromage sec, le lait de soja et le lait de soja fermenté, les jus de fruit-légume, les jus de fruit, les boissons de sport, le dessert, les bonbons, les formules pour nourrissons, les produits diététiques, l'alimentation animale et les compléments diététiques.

5. Produit alimentaire selon l'une quelconque des revendications 2 à 4, qui est fabriqué sous la forme d'un aliment instantané.

6. Composition pharmaceutique comprenant une quantité probiotiquement efficace d'une souche isolée de *Lactobacillus sp.* selon la revendication 1.

7. Composition pharmaceutique selon la revendication 6, où la composition est formulée en une forme de posologie orale choisie dans le groupe constitué par une solution, une émulsion, une poudre, un comprimé et une capsule.

8. Composition pharmaceutique selon la revendication 6 ou la revendication 7, où la composition est fabriquée en un médicament améliorant la digestion.

9. Composition pharmaceutique selon la revendication 6 ou la revendication 7, où la composition est fabriquée en un médicament pour abaisser le cholestérol sérique.

10. Utilisation d'une souche de *Lactobacillus sp.* selon la revendication 1 pour la fabrication d'un médicament pour le traitement ou la prévention des maladies gastro-intestinales ou pour abaisser le cholestérol sérique.

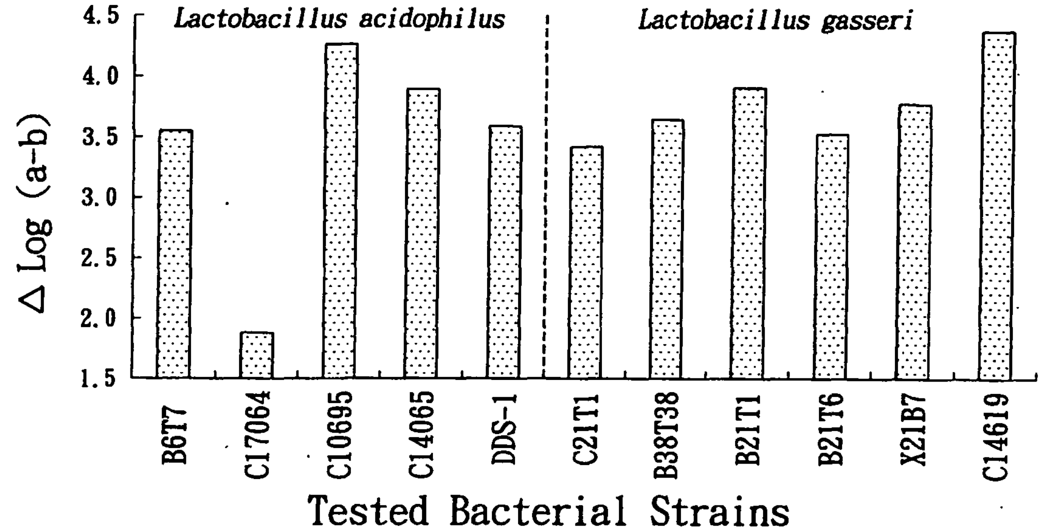
Lactobacillus acidophilus
Lactobacillus gasseri
4.5
4.0
3.5
3.0
2.5
2.0
1.5
Δ Log (a-b)
B6T7
C17064
C10695
C14065
DDS-1
C21T1
B38T38
B21T1
B21T6
X21B7
C14619
Tested Bacterial Strains

**FIG. 1**

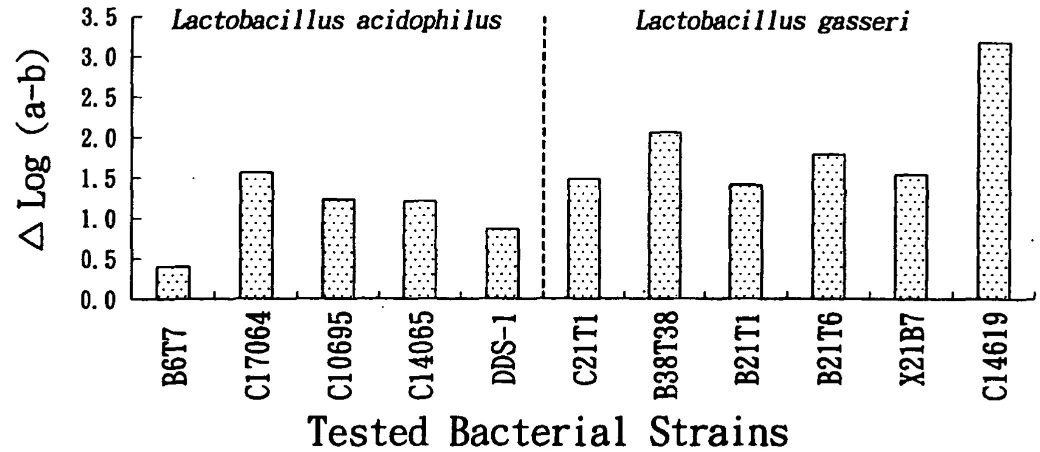
Lactobacillus acidophilus
Lactobacillus gasseri
3.5
3.0
2.5
2.0
1.5
1.0
0.5
0.0
Δ Log (a-b)
B6T7
C17064
C10695
C14065
DDS-1
C21T1
B38T38
B21T1
B21T6
X21B7
C14619
Tested Bacterial Strains

**FIG. 2**

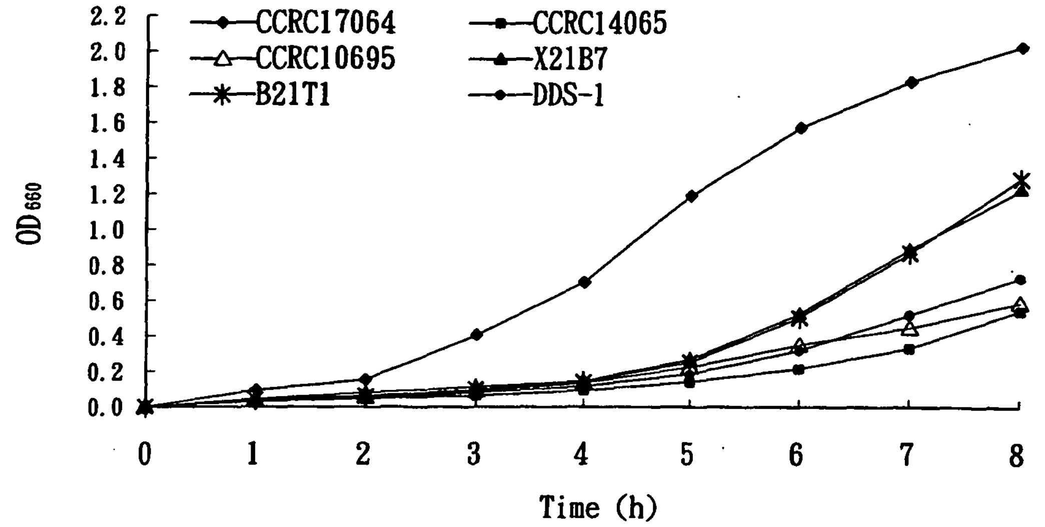
CCRC17064
CCRC14065
CCRC10695
X21B7
B21T1
DDS-1
OD 660
Time (h)

FIG. 3

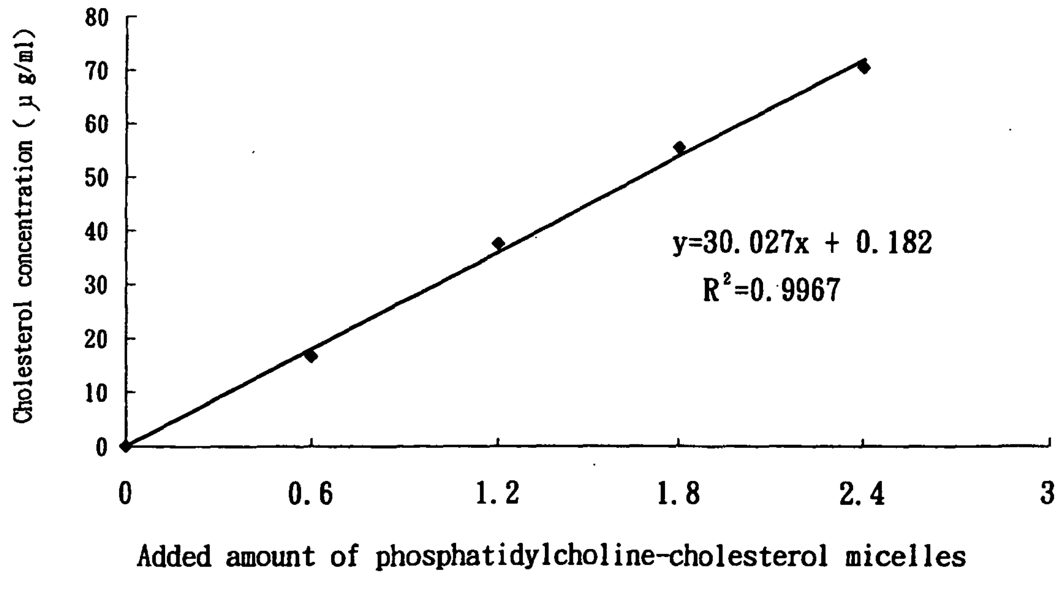
Cholesterol concentration ( μ g/ml)
y=30.027x + 0.182
R²=0.9967
Added amount of phosphatidylcholine-cholesterol micelles

FIG. 4

CAGGACGAAC GCTGGCGGCG TGCCTAATAC ATGCAAGTCG AGCGAGCTTG
CCTAGATGAA TTTGGTGCTT GCACCAAATG AAACTAGATA CAAGCGAGCG
GCGGACGGGT GAGTAACACG TGGGTAACCT GCCCAAGAGA CTGGGATAAC
ACCTGGAAAC AGATGCTAAT ACCGGATAAC AACACTAGAC GCATGTCTAG
AGTTTAAAAG ATGGTTCTGC TATCACTCTT GGATGGACCT GCGGTGCATT
AGCTAGTTGG TAAGGTAACG GCTTACCAAG GCAATGATGC ATAGCCGAGT
TGAGAGACTG ATCGGCCACA TTGGGACTGA GACACGGCCC AAACTCCTAC
GGGAGGCAGC AGTAGGGAAT CTTCCACAAT GGACGCAAGT CTGATGGAGC
AACGCCGCGT GAGTGAAGAA GGGTTTCGGC TCGTAAAGCT CTGTTGGTAG
TGAAGAAAGA TAGAGGTAGT AACTGGCCTT TATTTGACGG TAATTACTTA

FIG. 5

CAGGACGAAC GCTGGCGGCG TGCCTAATAC ATGCAAGTCG AGCGAGCTTG
CCTAGATGAA TTTGGTGCTT GCACCAAATG AAACTAGATA CAAGCGAGCG
GCGGACGGGT GAGTAACACG TGGGTAACCT GCCCAAGAGA CTGGGATAAC
ACCTGGAAAC AGATGCTAAT ACCGGATAAC AACACTAGAC GCATGTCTAG
AGTTTAAAAG ATGGTTCTGC TATCACTCTT GGATGGACCT GCGGTGCATT
AGCTAGTTGG TAAGGTAACG GCTTACCAAG GCAATGATGC ATAGCCGAGT
TGAGAGACTG ATCGGCCACA TTGGGACTGA GACACGGCCC AAACTCCTAC
GGGAGGCAGC AGTAGGGAAT CTTCCACAAT GGACGCAAGT CTGATGGAGC
AACGCCGCGT GAGTGAAGAA GGGTTTCGGC TCGTAAAGCT CTGTTGGTAG
TGAAGAAAGA TAGAGGTAGT AACTGGCCTT TATTTGACGG TAATTACTTA

FIG. 6

CAGGACGAAC GCTGGCGGCG TGCCTAATAC ATGCAAGTCG AGCGAGCTTG
CCTAGATGAA TTTGGTGCTT GCACCAAATG AAACTAGATA CAAGCGAGCG
GCGGACGGGT GAGTAACACG TGGGTAACCT GCCCAAGAGA CTGGGATAAC
ACCTGGAAAC AGATGCTAAT ACCGGATAAC AACACTAGAC GCATGTCTAG
AGTTTAAAAG ATGGTTCTGC TATCACTCTT GGATGGACCT GCGGTGCATT
AGCTAGTTGG TAAGGTAACG GCTTACCAAG GCAATGATGC ATAGCCGAGT
TGAGAGACTG ATCGGCCACA TTGGGACTGA GACACGGCCC AAACTCCTAC
GGGAGGCAGC AGTAGGGAAT CTTCCACAAT GGACGCAAGT CTGATGGAGC
AACGCCGCGT GAGTGAAGAA GGGTTTCGGC TCGTAAAGCT CTGTTGGTAG
TGAAGAAAGA TAGAGGTAGT AACTGGCCTT TATTTGACGG TAATTACTTA

FIG. 7

CAGGACGAAC GCTGGCGGCG TGCCTAATAC ATGCAAGTCG AGCGAGCTTG
CCTAGATGAT TTTGGTGCTT GCACTAAATG AAACTAGATA CAAGCGAGCG
GCGGACGGGT GAGTAACACG TGGGTAACCT GCCCAAGAGA CTGGGATAAC
ACCTGGAAAC AGATGCTAAT ACCGGATAAC AACACTAGAC GCATGTCTAG
AGTTTGAAAG ATGGTTCTGC TATCACTCTT GGATGGACCT GCGGTGCATT
AGCTAGTTGG TAAGGTAACG GCTTACCAAG GCAATGATGC ATAGCCGAGT
TGAGAGACTG ATCGGCCACA TTGGGACTGA GACACGGCCC AAACTCCTAC
GGGAGGCAGC AGTAGGGAAT CTTCCACAAT GGACGAAAGT CTGATGGAGC
AACGCCGCGT GAGTGAAGAA GGGTTTCGGC TCGTAAAGCT CTGTTGGTAG
TGAAGAAAGA TAGAGGTAGT AACTGGCCTT TATTTGACGG TAATTACTTA

# FIG. 8

CAGGACGAAC GCTGGCGGCG TGCCTAATAC ATGCAAGTCG AGCGAGCTTG
CCTAGATGAA TTTGGTGCTT GCACCAAATG AAACTAGATA CAAGCGAGCG
GCGGACGGGT GAGTAACACG TGGGTAACCT GCCCAAGAGA CTGGGATAAC
ACCTGGAAAC AGATGCTAAT ACCGGATAAC AACACTAGAC GCATGTCTAG
AGTTTAAAAG ATGGTTCTGC TATCACTCTT GGATGGACCT GCGGTGCATT
AGCTAGTTGG TAAGGTAACG GCTTACCAAG GCAATGATGC ATAGCCGAGT
TGAGAGACTG ATCGGCCACA TTGGGACTGA GACACGGCCC AAACTCCTAC
GGGAGGCAGC AGTAGGGAAT CTTCCACAAT GGACGCAAGT CTGATGGAGC
AACGCCGCGT GAGTGAAGAA GGGTTTCGGC TCGTAAAGCT CTGTTGGTAG
TGAAGAAAGA TAGAGGTAGT AACTGGCCTT TATTTGACGG TAATTACTTA

# FIG. 9

```
B6T7 CA - GACGAAC GCTGGCGGCG TGCCTAATAC ATGCAAGTCG AGCG - AC - CT
La   CAGGACGAAC GCTGGCGGCG TGCCTAATAC ATGCAAGTCG AGCG - AG - CT
Lp   CAGGACGAAC GCTGGCGGCG TGCCTAATAC ATGCAAGTCG AACGAACTCT
     . . * . . . . . . . . . . . . . . . . . . . . . . . . . . . . . . . . . . . . . . . * . . * . * * . .

B6T7 - GAACCAACA GAT - - TCACT TCGGTGA - TG ACGTTGGGAA CGCGAGCGGC
La   - GAACCAACA GAT - - TCACT TCGGTGA - TG ACGTTGGGAA CGCGAGCGGC
Lp   GGTATTGATT GGTGCTTGCA TC - ATGATTT ACATT - TG - A - GTGAGTGGC
     * . * . * * * . * * . * . * * . * * . * . . * * . . . * . * . . * . . * * . * . * . * . . . * . . .

B6T7 GGATGGGTGA GTAACACGTG GGGAACCTGC CCCAT - AGTC TGGGATACCA
La   GGATGGGTGA GTAACACGTG GGGAACCTGC CCCAT - AGTC TGGGATACCA
Lp   GAACTGGTGA GTAACACGTG GGAAACCTG - CCCAGAAGCG GGGGATAACA
     . * . * * . . . . . . . . . . . . . . . . * . . . . . . * . . . . * * . . * * * . . . . . . * . .

B6T7 CTTGGAAACA GGTGCAATA CCGGATAAGA AAGCAGATGC CATGATCAGC
La   CTTGGAAACA GGTGCAATA CCGGATAAGA AAGCAGATGC CATGATCAGC
Lp   CCTGGAAACA GATGCTAATA CCGCATAACA ACTTGGACCG CATGGTCCGA
     . * . . . . . . . . * . . . . . . . . . . . * . . . . * . . * * * * . . * . . . . . . . * . . * . *

B6T7 TTATAAAAGG CGGCGTAAGC TGTCGCTATG GGATGGCCCC GCGGTGCATT
La   TTATAAAAGG CGGCGTAAGC TGTCGCTATG GGATGGCCCC GCGGTGCATT
Lp   G - TTGAAAGA TGGCTTCGGC TATCACTTTT GGATGGTCCC GCGGCGTATT
     * * * . * . . . . * . * . . . . . . . . . * . . . . . . . . . . . . * . . . . . * . . * * * * . . * . .

B6T7 AGCTAGTTGG TAGGGTAACG GCCTACCAAG GCAATGATGC ATAGCCGAGT
La   AGCTAGTTGG TAGGGTAACG GCCTACCAAG GCAATGATGC ATAGCCGAG -
Lp   AGCTAGATGC TGGGGTAACG GCTCACCATG GCAATGATAC GTAGCCGA - C
     . . . . * . . * . * * * * . * . . . . * * . . . * . * * * . . * . . * . . * . * . . . . . . * . . .

B6T7 TTGAGAGACT GATCCGGCCA CATTGGGACT GAGACACGGC CCAAACTCCT
La   TTGAGAGACT GAT - CGGCCA CATTGGGACT GAGACACGGC CCAAACTCCT
Lp   CTGAGAGGGT AAT - CGGCCA CATTGGGACT GAGACACGGC CCAAACTCCT
     * . . . . . . * * . * . . * . . . . . . . . . . . . . . . . . . . . . . . . . . . . . . . . . . .

B6T7 ACGGGAGGGC AAGCAGTAGG GAATCCTCCA CAATGGACCA AAGTCCTGAT
La   ACGGGA - GGC - AGCAGTAGG GAATCTTCCA CAATGGACGA AAGT - CTGAT
Lp   ACGGGA - GGC - AGCAGTAGG GAATCTTCCA CAATGGACGA AAGT - CTGAT
     . . . . . . * . . . * . . . . . . . . . . . . . . * . . . . . . . . . . . . . * . . . . . . * . . . . .

B6T7 GGAGCAACGC CCCGTGAGTT GAAGAA - GTT TTCGGATCGT AAAGCCCTGT
La   GGAGCAACGC CGCGTGAG - T GAAGAAGGTT TTCGGATCGT AAAGCTCTGT
Lp   GGAGCAACGC CGCGTGAG - T GAAGAAGGGT TTCGGCTCGT AAAACTCTGT
     . . . . . . . . . . . * . . . . . . . * . . . . . . . * . * . . . . . . * . . . . . . . . * . * . . . .

B6T7 TGTTGGTGAA GAAGGATA - G AGGTAAGAAC TG - GCCTTTA TTTGACCGTA
La   TGTTGGTGAA GAAGGATA - G AGGTAGTAAC TG - GCCTTTA TTTGACGGTA
Lp   TGTTAAAGAA GAA - CATATC TGAGAGTAAC TGTTCAGGTA - TTGACGGTA
     . . . . * * * . . . . . . . . * * . . . . * * * . * * . * * . . . . . . * * . * * * . . * . . . . . . * . . .
```

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0792586 A
- US 4839281 A
- US 5032399 A
- US 5516684 A
- US 5707854 A
- US 5711977 A


### Non-patent literature cited in the description


- **W.C. FRAZIER ; D.C. WESTHOFF.** Food Microbiology. McGraw-Hill, Inc, 1978
- **O'SULLIVAN et al.** *Trends in Food Sci. Technol.,* 1992, vol. 3, 309-314
- Probiotics: prospects of use in opportunistic infections. Old Herbom University Seminar Monograph, 1995, 1
- **ELI METCHNIKOFF.** The Prolongation Of Life. The Knickerbocker Press, 1908
- **J. DENTER ; B. BISPING.** *Int. J. Food Microbiol.,* 1994, vol. 22, 23-31
- **HOSE, H. ; SOZZI, T.** *J. Chem. Technol. And Biotech.,* 1991, vol. 51, 540-544
- **H. MAJAMAA et al.** *Journal of Pediatric Gastro-enterology and Nutrition,* 1995, vol. 20, 333-338
- **E.J. SCHIFFRIN et al.** *Am. J. Clin. Nutr.,* 1997, vol. 66, 515S-520S
- **KANNEL et al.** *Ann. Intern. Med.,* 1979, vol. 90, 85-91
- **PEKKANEN et al.** *New England J. Med.,* 1990, vol. 322, 1700-1707
- *Am. J. Clin. Nutr.,* 1974, vol. 27, 464-469
- **K.K. GRUNEWALD.** *J. of Food Science,* 1982, vol. 47, 2078-2079
- **DANIELSON et al.** *J. Anim. Sci.,* 1989, vol. 67, 966-974
- **A.S. AKALIN.** *J. Dairy Sci.,* 1997, vol. 80, 2721-2725
- **DE SMET et al.** *British J. of Nutrition,* 1998, vol. 79, 185-194
- **S.E. GILLILAND et al.** *Appl. Environ. Microbiol,* 1985, vol. 49, 377-381
- **NOH et al.** *J. Dairy Sci.,* 1997, vol. 82, 3107-3113
- **F.A.M. KALVER ; R. VAN DER MEER.** *Appl. Environ. Microbiol.,* 1993, vol. 59, 1120-1124
- **M.M. BRASHEARS ; S.E. GILILLAND.** Influences of pH during growth on removal of cholesterol from MRS broth. *Lactobacillus casei and Lactobacillus acidophilus, Animal Science Research Report,* 1997, 32-37, http://www.ansi.okstate.edu/research/1997rr/006.htm
- **ZHANG JIA-CHENG et al.** The research of cholesterol elimination in food by lactic acid bacteria - the screening of lactic acid bacteria species (strains. *Food Science (P.R.O.C,* 1998, vol. 19, 20-22
- **USMAN ; HOSONO.** *J. Dairy Sci.,* 1999, vol. 82, 243-248
- *J. Dairy Sci.,* 1999, vol. 82, 2536-2542
- **LUCHANSKY et al.** *J. Dairy Sci.,* vol. 74, 3293-3302
- **S.E. GILLILAND ; D.K. WALKER.** *J. Dairy Sci.,* 1990, vol. 73, 905-911
- *Potrav. Védy,* vol. 14 (3), 165-174
- **S. RAZIN et al.** *Biochimica Biophysica Acta,* 1980, vol. 598, 628-640
- **S.E. GILLILAND et al.** *Appl. Environ. Microbiol.,* 1985, vol. 49, 377-381
- **F.A.M. KALVER ; R. VAN DER MEER.** *Appl. Environ. Microbiol,* 1993, vol. 59, 1120-1124
- **D.O. NOH et al.** *J. Dairy Sci.,* 1997, vol. 82, 3107-3113
- **D.K. WALKER ; S.E. GILLILAND.** *J. Dairy Sci.,* 1993, vol. 76, 956-961
- *Int. J. Syst. Bacterial.,* 1980, vol. 30, 601
- **E. LAUER ; O. KANDLER.** *Bakteriol. Parasitenkd. Infektionskr. Hyg. Abt. 1 Orig. Reihe C,* 1980, vol. 1, 75-78
- **J.E. HOLCOMBE et al.** *Cult. Dairy Prod. J.,* 1991, vol. 26 (3), 4-5
- **S.E. GILLILAND et al.** *J. Dairy Sci.,* 1984, vol. 67, 3045-3051
- **P. MARTEAU et al.** *J. Dairy Sci.,* 1997, vol. 80, 1031-1037
- **S.E. GILLILAND ; D.K. WALKER.** *J. Dairy Sci.,* 1990, vol. 73, 905-911, J. Dairy Sci.
- **D.K. WALKER ; S.E. GILLILAND.** *J. Dairy Sci.,* 1993, vol. 76, 956-961, J. Dairy Sci.
- **HUANG, C. ; T.E. THOMOPSON.** *Methods Enzymol,* 1974, vol. 32, 485-489
- **L.L. RUDEL ; M.D. MORRIS.** *Notes on Methodology,* 1973, vol. 14, 364-366

- Regular, nonsporing Gram-positive rods and Cocci. **O. KANDLER ; N. WEISS.** Bergey's Manual of Systematic Bacteriology. The Williams & Wilkins Co, 1986, vol. II, 1208-1234
- **G. H. FLEET et al.** *Appl. Environ. Microbiol.,* 1984, vol. 48, 1034-1038
- **M. ROGOSA et al.** Method for coding data on microbial strains for computers (edition AB. *Int. J. Syst. Bacteriol.,* 1971, vol. 21, 1A-184A
- **ROSENBLUM et al.** *Nucleic Acid Res.,* 1997, vol. 25, 4500-4504
- MicroSeq™ Reference Manual. PE CO, 1999
- **G. KLEIN et al.** *International Journal of Food Microbiology.,* 1998, vol. 41, 103-125